(19) Europäisches Patentamt
European Patent Office
Office européen des brevets

(11) **EP 0 815 861 B1**

(12) **EUROPÄISCHE PATENTSCHRIFT**

(45) Veröffentlichungstag und Bekanntmachung des Hinweises auf die Patenterteilung:
**19.09.2001 Patentblatt 2001/38**

(21) Anmeldenummer: **97109984.1**

(22) Anmeldetag: **19.06.1997**

(51) Int Cl.⁷: **A61K 31/505**, A61K 31/44, A61K 31/40, A61K 31/63, A61K 31/18, C07D 213/76, C07D 209/40, C07D 239/50, C07D 239/48, C07C 311/44, C07C 311/21

(54) **Sulfonamide und ihre Verwendung**

Sulphonamides and their use

Sulphonamides et leur utilisation

(84) Benannte Vertragsstaaten:
**AT BE CH DE DK ES FI FR GB GR IE IT LI LU MC NL PT SE**

(30) Priorität: **28.06.1996 EP 96110462**
**01.04.1997 EP 97105363**

(43) Veröffentlichungstag der Anmeldung:
**07.01.1998 Patentblatt 1998/02**

(73) Patentinhaber: **F. HOFFMANN-LA ROCHE AG**
**4070 Basel (CH)**

(72) Erfinder:
- **Bös, Michael**
  **4310 Rheinfelden (CH)**
- **Godel, Thierry**
  **4056 Basel (CH)**
- **Riemer, Claus**
  **79110 Freiburg (DE)**
- **Sleight, Andrew**
  **68400 Riedisheim (FR)**

(74) Vertreter: **Poppe, Regina et al**
**F.Hoffmann-La Roche AG**
**Patent Department(PLP),**
**124 Grenzacherstrasse**
**4070 Basel (CH)**

(56) Entgegenhaltungen:
**WO-A-92/14456**

Bemerkungen:
Die Akte enthält technische Angaben, die nach dem Eingang der Anmeldung eingereicht wurden und die nicht in dieser Patentschrift enthalten sind.

EP 0 815 861 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Verbindungen der allgemeinen Formel I beziehungsweise ihre Verwendung zur Herstellung von Medikamenten zur Bekämpfung oder Verhütung von zentralnervösen Störungen,

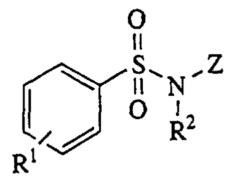

I

worin

Z eine substituierte Phenyl-, substituierte Pyridyl-, substituierte Pyrimidyl- oder substituierte Indolylgruppe der Formeln a-e

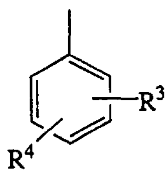 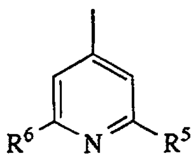 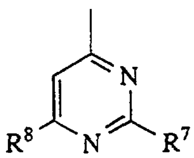 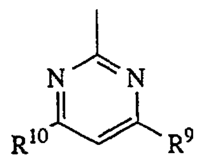 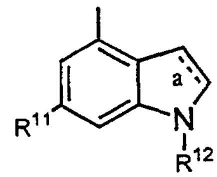

a , b , c , d und e

| | |
|---|---|
| $R^1$ | Wasserstoff, Amino, $C_{1-7}$-Alkylamino, $C_{1-7}$-Dialkylamino, $C_{1-7}$-Alkyl, Halogen oder Trifluormethyl; |
| $R^2$ | Wasserstoff oder $C_{1-7}$-Alkyl; |
| $R^3$ | Wasserstoff, Amino, $C_{1-7}$-Alkylamino, $C_{1-7}$-Dialkylamino, $C_{1-7}$-Alkyl, $CF_3$, $C_{1-7}$-Alkoxyoder Halogen; |
| $R^4$ | Amino, $C_{1-7}$-Alkylamino, $C_{1-7}$-Dialkylamino, $C_{1-7}$-Alkyl, $C_{1-7}$-Alkoxy oder Halogen; |
| $R^5$ | Wasserstoff, $C_{1-7}$-Alkylamino, di- $C_{1-7}$-Alkylamino, $C_{1-7}$-Alkoxy oder Halogen; |
| $R^6$ | $C_{1-7}$-Alkyl, $C_{1-7}$-Alkylamino, di- $C_{1-7}$-Alkylamino, $C_{1-7}$-Alkoxy, Halogen oder $CF_3$; |
| $R^7$ | Amino, $C_{1-7}$-Alkylamino, di- $C_{1-7}$-Alkylamino, $C_{1-7}$-Alkoxy, $C_{1-7}$-Alkylsulfanyl, Mercapto, Pyrrolidin-1-yl oder Azetidin-1-yl; |
| $R^8$ | Amino, $C_{1-7}$-Alkylamino, di- $C_{1-7}$-Alkylamino, Benzylamino, $C_{1-7}$-Alkoxy, $C_{1-7}$-Alkylsulfanyl, Halogen, Pyrrolidin-1-yl oder Azetidin-1-yl; |
| $R^9$ und $R^{10}$ | unabhängig voneinander $C_{1-7}$-Alkoxy oder $C_{1-7}$-Alkylamino; |
| $R^{11}$ | Wasserstoff oder Halogen; |
| $R^{12}$ | Wasserstoff oder $C_{1-7}$-Alkyl, und |
| a | gegebenenfalls eine Doppelbindung bedeuten |

mit der Massgabe, dass $R^7$ und $R^8$ nicht gleichzeitig Methoxy bedeuten, sowie deren pharmazeutisch annehmbare Salze.

Die Formel I umfasst bekannte und noch neue Verbindungen.

Sowohl die an sich bereits bekannten als auch die neuen Verbindungen besitzen überraschenderweise eine selektive Affinität zu 5HT-6 Rezeptoren. Sie eignen sich somit zur Behandlung oder Verhütung von zentralnervösen Störungen wie beispielsweise von Psychosen, Schizophrenie, manischen Depressionen ( Bryan L. Roth et al., J. Pharmacol. Exp. Ther., **268**, Seite 1403-1410 (1994)), Depressionen ( David R. Sibley et al., Mol. Pharmacol., **43**, Seite 320-327 (1993)), neurologischen Störungen (Anne Bourson et al., J. Pharmacol. Exp. Ther., 274, Seite 173-180 (1995); R. P.Ward et al., Neuroscience, 64, Seite 1105-1110 (1995)), Gedächtnisstörungen, der Parkinson'schen Krankheit, der amyotrophen Lateralsklerose, der Alzheimer'schen Krankheit und der Huntington'sche Krankheit (Andrew J. Sleight et al., Neurotransmissons, **11**, Seite 1-5 (1995) ).

Gegenstand der vorliegenden Erfindung ist die Verwendung von Verbindungen der allgemeinen Formel I und von deren pharmazeutisch anwendbaren Salzen zur Herstellung von Medikamenten zur Behandlung oder Verhütung von Krankheiten der oben erwähnten Art, die neuen Verbindungen der Formeln $Ia_1$, $Ia_2$, $Ib_1$, $Ib_2$, $Ic_1$, $Ic_2$, $Id_1$, $Id_2$ und Ie oder deren pharmazeutisch anwendbare Salze als solche oder zur Anwendung als therapeutische Wirkstoffe, die

Herstellung dieser neuen Verbindungen, Arzneimittel, enthaltend eine neue oder bekannte Verbindung der Formel I oder pharmazeutisch anwendbare Salze davon sowie die Herstellung solcher Arzneimittel.

Verbindungen der Formel I, worin Z ein substituierter Phenylrest ist, sind z. B. von R. Behnisch et al. in Chemische Berichte, **81**, No. 4, Seite 297-306, (1947) beschrieben, beispielsweise Verbindungen mit Halogen-Substituenten am Phenylrest wie 4-Amino-N-(3,5-dichlor-phenyl)-benzolsulfonamid oder 4-Amino-N-(3,5-dibrom-phenyl)-benzolsulfonamid, oder Verbindungen mit Methyl-oder Methoxy- Substituenten am Phenylrest wie 4-Amino-N-(3,5-dimethyl-phenyl)-benzolsulfonamid oder 4-Amino-N-(3,5-dimethoxy-phenyl)-benzolsulfonamid. Die hier beschriebenen Sulfonamide besitzen eine Wirkung gegen Malaria.

J. L. Fedrick et al. beschreiben in Journal of Org. Chem., **26**, Seite 4715-4716, (1961) Fluorsulfanilanilide wie beispielsweise die Verbindung 4-Amino-N-(3,5-difluor-phenyl)-benzolsulfonamid sowie deren Herstellung und Verwendung als antibakterielle Mittel.

Ferner beschreibt J. K. Seydel in Mol. Pharmacol., **2**, Seite 259-265, (1966) die in vitro Aktivität einer Reihe von Sulfonamiden gegen E. coli .

Bergeim et al. beschreiben in Journal of the American Chem. Soc., **69**, Seite 583-587, (1947) die Herstellung einiger Aminosulfanilaniside als mögliche Antimalariamittel wie beispielsweise die Verbindung 4-Amino-N-(3-methoxy-phenyl)-benzolsulfonamid.

Keine der oben aufgeführten Veröffentlichungen erwähnen, dass Phenyl-benzolsulfonamide auch als wirksame Verbindungen zur Behandlung von neurologischen Störungen geeignet sein könnten.

Verbindungen der Formel I, worin Z ein substituierter Pyridinrest ist, sind beispielsweise von R. Urban et al. in Helvetia Chimica Acta, **47**, Seite 363-377, (1964) beschrieben, beispielsweise die Verbindungen 4-Amino-N-(4,6-dimethoxy-pyridin-2-yl)-benzolsulfonamid, 4-Amino-N-(2-methoxy-6-brom-pyridin-3-yl)-benzolsulfonamid, 4-Amino-N-(2-methoxy-6-methylpyridin-4-yl)-benzolsulfonamid oder 4-Amino-N-(2,6-dimethoxy-pyridin-4-yl)-benzolsulfonamid. Beschrieben wird die Herstellung von Aminomethoxypyridinen und Sulfanilamiden und deren antibakterielle Eigenschaften. Auch in dieser Veröffentlichung findet sich keinerlei Hinweis auf eine mögliche Wirkung gegen neurologische Störungen.

Verbindungen der Formel I, worin Z ein substituierter Pyrimidinrest ist, sind z. B. von Bretschneider et al. in Monatshefte für Chemie, **92**, Seite 183-192, (1961) beschrieben. Es wird die Herstellung von 2,6-disubstituierten 4-Sulfanil-amidopyrimidinen beschrieben, beispielsweise von der Verbindung 4-Amino-N-(2,6-diethylsulfanyl-pyrimidin-4-yl)-benzolsulfonamid. In Monatshefte für Chemie, **95**, Seite 207-213, (1964) beschreiben Bretschneider et al. weitere Synthesen zur Herstellung von 6-Sulfanilamido-2,4-dimethoxypyrimidin.

W. Baker et al. beschreiben in Journal of the American Chem. Soc., **69**, Seite 3072-3078, (1947) die Herstellung von substituierten Sulfanilamidopyrimidinen sowie deren mögliche Verwendung als antibakterielle Mittel, wie beispielsweise die Verbindung 4-Amino-N-(4,6-dimethoxy-pyrimidin-2-yl)-benzolsulfonamid.

In der französischen Patentanmeldung FR 1 383 287 sind Sulfanilderivate von 2,6-disubstituierten Pyrimidinen beschrieben, wie beispielsweise die Verbindung 4-Amino-N-(2,6-bis-dimethylaminopyrimidin-4-yl)-benzolsulfonamid.

Keine dieser Veröffentlichungen erwähnt, dass Sulfonamide, die einen Pyrimidinrest tragen, auch als wirksame Verbindungen zur Behandlung von neurologischen Störungen geeignet sein könnten.

In der internationalen Patentanmeldung WO 92/14456 wird ganz allgemein die Verwendung von Verbindungen, die eine primäre Amingruppe tragen, zur Behandlung von neurologischen Störungen beschrieben. Eine Vielzahl verschiedener Verbindungsgruppen, wie zum Beispiel p-Aminobenzoesäuren, p-Aminophenylessigsäuren, Aminonikotinsäure, 2,3-diaminopropionsäure u.s.w. werden genannt. In dieser Aufzählung verschiedener Verbindungsgruppen werden u.a. auch Sulfanilamide und 1-Amino substituierte Derivate von Sulfanilamiden ($p-H_2N-C_6H_4-SO_2NHR$) genannt. Die spezifisch genannte Verbindung 4-Amino-N-(2,6-dimethoxy-pyrimidin-4-yl)-benzolsulfonamid wird mit Hilfe eines Disclaimers von der Verwendung der Verbindungen der Formel I zur Behandlung oder Verhütung von Krankheiten der oben erwähnten Art Störungen ausgeschlossen.

Die Verbindungen der Formel I umfassen folgende Gruppen:

a) Anilide der Formel Ia,

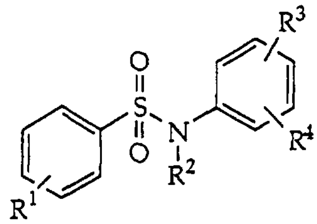

Ia

worin $R^1$, $R^2$, $R^3$ und $R^4$ die in Formel I angegebenen Bedeutungen haben;

b) Verbindungen der Formel Ib,

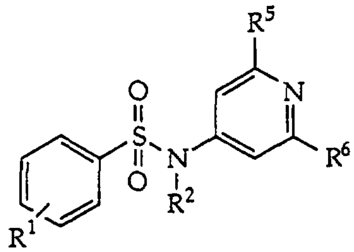

I b

worin $R^1$, $R^2$, $R^5$ und $R^6$ die in Formel I angegebenen Bedeutungen haben,

c) Verbindungen der Formeln Ic und Id,

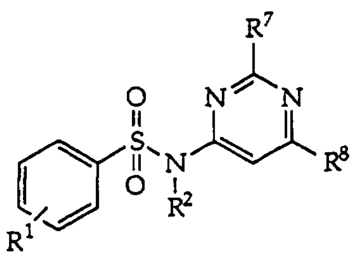

I c

,

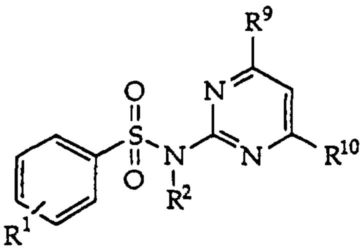

I d

worin $R^1$, $R^2$, $R^7$, $R^8$, $R^9$ und $R^{10}$ die in Formel I angegebenen Bedeutungen haben, und

d) Verbindungen der Formeln Ie

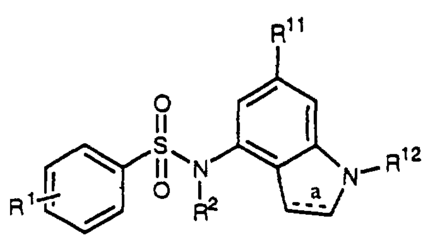

I e

worin $R^1$, $R^2$, $R^{11}$, $R^{12}$ und a die in Formel I angegebene Bedeutung haben.

Die folgenden Verbindungen der Formeln Ia, Ib, Ic, Id und Ie sind für eine Verwendung der oben beschriebenen Art besonders bevorzugt:

4-Amino-N-(2,6-bis-methylamino-pyrimidin-4-yl)-benzolsulfonamid;

4-Amino-N-(6-ethylamino-2-methylamino-pyrimidin-4-yl)-benzolsulfonamid;

4-Amino-N-(2-dimethylamino-6-methylamino-pyrimidin-4-yl)-benzolsulfonamid;

4-Amino-N-(2-dimethylamino-6-ethylamino-pyrimidin-4-yl)-benzolsulfonamid;

4-Amino-N-(2,6-bis-methylamino-pyrimidin-4-yl)-N-methyl-benzolsulfonamid;

4-Amino-N-(2-azetidin-1-yl-6-methylamino-pyrimidin-4-yl)-benzolsulfonamid;

4-Amino-N-(2-azetidin-1-yl-6-ethylamino-pyrimidin-4-yl)-benzolsulfonamid;

4-Amino-N-(6-methylamino-2-pyrrolidin-1-yl-pyrimidin-4-yl)-benzolsulfonamid;

4-Amino-N-(2-brom-6-methylamino-pyridin-4-yl)-benzolsulfonamid;

4-Amino-N-(2,6-bis-methylamino-pyridin-4-yl)-benzolsulfonamide;

4-Amino-N-(2-ethylamino-6-methylamino-pyridin-4-yl)-benzolsulfonamid;

4-Amino-N-(2-dimethylamino-6-methylamino-pyridin-4-yl)-benzolsulfonamid.

4-Amino-N-(2,6-bis-ethylamino-pyridin-4-yl)-benzolsulfonamid;

N-(2,6-Bis-methylamino-pyrimidin-4-yl)-3-chlor-benzolsulfonamid;

N-(2,6-Bis-methylamino-pyrimidin-4-yl)-3-trifluormethyl-benzolsulfonamid;

4-Amino-N-(2-methyl-6-methylamino-pyridin-4-yl)-benzolsulfonamid;

4-Amino-N-(3,5-dimethoxy-phenyl)-benzenesulfonamide;

4-Amino-N-(3,5-dichloro-phenyl)-benzenesulfonamide;

4-Amino-N-(3,5-dibromo-phenyl)-benzenesulfonamide und

4-Amino-N-(1H-indol-4-yl)-benzolsulfonamid.

Der in der vorliegenden Beschreibung verwendete Ausdruck " $C_{1-7}$-Alkyl" bezeichnet Reste von 1 bis 7, vorzugsweise von 1 bis 4 Kohlenstoff-Atomen, wie beispielsweise Methyl, Ethyl, n-Propyl, i-Propyl, n-Butyl, i-Butyl und t-Butyl.

Der Ausdruck " $C_{1-7}$-Alkoxy" bezeichnet einen über ein Sauerstoffatom gebundenen $C_{1-7}$-Alkylrest im Sinne der vorstehenden Definition, wie beispielsweise Methoxy, Ethoxy, n-Propoxy, i-Propoxy, n-Butoxy, i-Butoxy und t-Butoxy.

Der Ausdruck" $C_{1-7}$-Alkylamino" bezeichnet einen über eine NH-Gruppe gebundenen $C_{1-7}$-Alkylrest im Sinne der vorstehenden Definition, wie beispielsweise Methylamino und Ethylamino.

Der Ausdruck "di- $C_{1-7}$-Alkylamino" bezeichnet zwei gleiche oder verschiedene über ein Stickstoffatom gebundene $C_{1-7}$-Alkylreste im Sinne der vorstehenden Definition, wie beispielsweise Dimethylamino, Diethylamino oder Methyl-ethyl-amino.

Der Ausdruck " $C_{1-7}$-Alkylsulfanyl" bezeichnet einen über ein Schwefelatom gebundenen $C_{1-7}$-Alkylrest im Sinne der vorstehenden Definition, wie beispielsweise Methylsulfanyl ($-S-CH_3$) und Ethylsulfanyl ($-S-CH_2CH_3$).

Der Ausdruck "Halogen " umfasst Fluor, Chlor, Brom und Jod.

In bezug auf Formel Ia sind die folgenden bekannten Verbindungen für die Anwendung als therapeutische Wirkstoffe bevorzugt bzw. besonders bevorzugt:

4-Amino-N-(3,5-dimethoxy-phenyl)-benzolsulfonamid,

4-Amino-N-(3,5-dichlor-phenyl)-benzolsulfonamid,

4-Amino-N-(3,5-dibrom-phenyl)-benzolsulfonamid.,

4-Amino-N-(3,5-dimethyl-phenyl)-benzolsulfonamid,

4-Amino-N-(3- methoxy-phenyl)-benzolsulfonamid.

Neue Verbindungen der Formel Ia sind Verbindungen der Formeln $Ia_1$ und $Ia_2$,

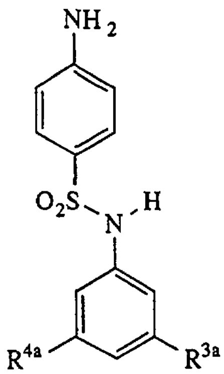

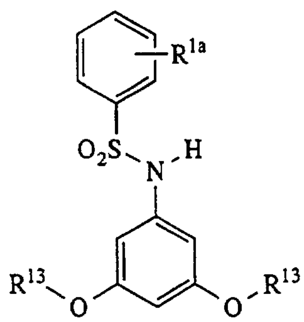

$Ia_1$ , $I a_2$

worin

$R^{1a}$ 3-Trifluormethyl, 3-Halogen oder 4-Halogen;
$R^{3a}$ Wasserstoff, Halogen, $C_{1-7}$-Alkoxy, Amino oder $C_{1-7}$-Alkylamino;
$R^{4a}$ Amino oder $C_{1-7}$-Alkylamino und
$R^{13}$ $C_{1-7}$-Alkyl bedeuten,

mit der Einschränkung, dass wenn $R^{4a}$ Amino bedeutet, $R^{3a}$ von Wasserstoff verschieden ist, sowie deren pharmazeutisch annehmbare Salze.

Bevorzugte neue Verbindungen der Formel $Ia_1$, worin

$R^{3a}$ Wasserstoff, Amino oder Methylamino;
$R^{4a}$ Amino oder Methylamino bedeuten,

und deren Herstellung sind in den Beispielen 34-36 beschrieben.

Beispiel 37 beschreibt die Herstellung einer neuen Verbindung der Formel $Ia_2$, worin $R^{1a}$ 3-Trifluormethyl und $R^{13}$ Methyl bedeutet.

In bezug auf Formel Ib sind die folgenden bekannten Verbindungen für die Anwendung als therapeutische Wirkstoffe bevorzugt:

4-Amino-N-(2,6-dimethoxy-pyridin-4-yl)-benzol-sulfonamid,

4-Amino-N-(2-methoxy-6-methyl-pyridin-4-yl)-benzolsulfonamid.

Neue Verbindungen der Formel Ib sind Verbindungen der Formeln $Ib_1$ und $I b_2$,

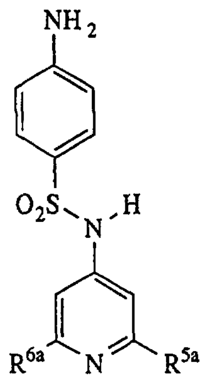

Ib$_1$ ,

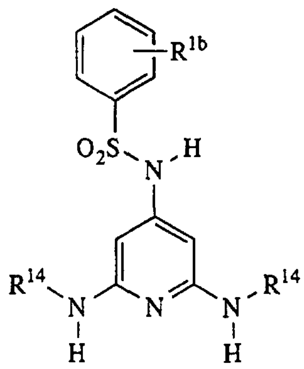

Ib$_2$

worin

- $R^{1b}$ 4-Halogen, 3-Halogen oder 3-Trifluormethyl;
- $R^{5a}$ Wasserstoff, $C_{1-7}$-Alkylamino, di- $C_{1-7}$-Alkylamino oder Halogen;
- $R^{6a}$ $C_{1-7}$-Alkyl, $CF_3$, $C_{1-7}$-Alkylamino, di- $C_{1-7}$-Alkylamino, oder Halogen und
- $R^{14}$ $C_{1-7}$-Alkyl bedeuten,

mit der Einschränkung, dass wenn $R^{6a}$ Halogen bedeutet, $R^{5a}$ von Wasserstoff verschieden ist, sowie deren pharmazeutisch annehmbare Salze.

Bevorzugte neue Verbindungen der Formel Ib$_1$, worin

- $R^{5a}$ Wasserstoff, Methylamino, Ethylamino, di-Methylamino, Chlor oder Brom und
- $R^{6a}$ Methyl, Methylamino, Ethylamino, di-Methylamino oder Brom bedeuten,

mit der Einschränkung, dass wenn $R^{6a}$ Brom bedeutet, $R^{5a}$ von Wasserstoff verschieden ist,
und deren Herstellung sind in den Beispielen 38 - 47, 51 und 53 beschrieben.

Bevorzugte neue Verbindungen der Formel Ib$_2$, worin

- $R^{1b}$ 4-Chlor, 3-Chlor oder 3-Trifluormethyl und
- $R^{14}$ Methyl bedeuten,

und deren Herstellung sind in den Beispielen 48 - 50 beschrieben.

Neue Verbindungen der Formel Ic sind Verbindungen der Formeln Ic$_1$und Ic$_2$,

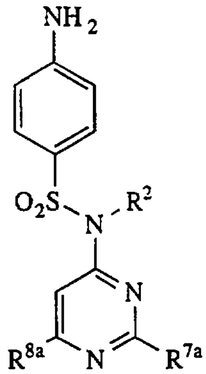 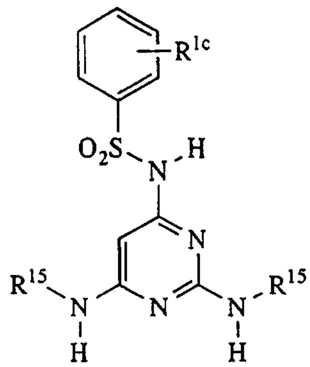

Ic$_1$ , Ic$_2$

worin

- $R^{1c}$ Wasserstoff, 4-Halogen, 4- $C_{1-7}$-Alkyl, 3-Halogen, 3- Trifluormethyl;
- $R^2$ Wasserstoff oder $C_{1-7}$-Alkyl;
- $R^{7a}$ Amino, $C_{1-7}$-Alkylamino, di- $C_{1-7}$-Alkylamino, Mercapto, Pyrrolidin-1-yl oder Azetidin-1-yl;
- $R^{8a}$ Amino, $C_{1-7}$-Alkylamino, di- $C_{1-7}$-Alkylamino, Benzylamino, $C_{1-7}$-Alkoxy, Pyrrolidin-1-yl oder Azetidin-1-yl und
- $R^{15}$ $C_{1-7}$-Alkyl bedeuten,

mit der Einschränkung, dass wenn $R^{7a}$ di- $C_{1-7}$-Alkylamino bedeutet, $R^{8a}$ von $C_{1-7}$-Alkoxy und von di- $C_{1-7}$-Alkylamino verschieden ist,
sowie deren pharmazeutisch annehmbare Salze.

Bevorzugte neue Verbindungen der Formel $Ic_1$, worin

- $R^2$ Wasserstoff oder Methyl;
- $R^{7a}$ Amino, Methylamino, Ethylamino, Propylamino, iso-Propylamino, Dimethylamino, Mercapto, Pyrrolidin-1-yl oder Azetidin-1-yl;
- $R^{8a}$ Amino, Methylamino, Ethylamino, Propylamino, iso-Propylamino, Dimethylamino, Benzylamino, Methoxy, Pyrrolidin-1-yl oder Azetidin-1-yl bedeuten,

mit der Einschränkung, dass, wenn $R^{7a}$ Dimethylamino bedeutet, $R^{8a}$ von Dimethylamino und Methoxy verschieden ist, und deren Herstellung sind in den Beispielen 1 - 25 beschrieben.

Bevorzugte neue Verbindungen der Formel $Ic_2$, worin

- $R^{1c}$ Wasserstoff, 4-Fluor, 4-Chlor, 4-Methyl, 4-tert. Butyl, 3-Chlor, 3-Trifluormethyl und
- $R^{15}$ Methyl bedeuten,

und deren Herstellung sind in den Beispielen 27 - 33 beschrieben.

Neue Verbindungen der Formel Id sind Verbindungen der Formeln $Id_1$ und $Id_2$,

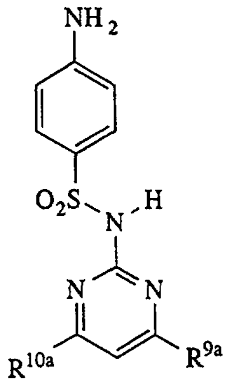

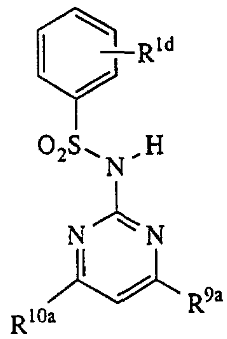

Id$_1$ , Id$_2$

worin

$R^{1d}$ 3-Trifluormethyl, 4-Trifluormethyl, 3-Halogen oder 4-Halogen und
$R^{9a}$ und $R^{10a}$ $C_{1-7}$-Alkylamino bedeuten,

sowie deren pharmazeutisch annehmbare Salze.

Beispiel 26 beschreibt die Herstellung einer neuen Verbindung der Formel I d$_1$, worin $R^{9a}$ und $R^{10a}$ Methylamino bedeuten.

Verbindungen der Formel Ie sind ebenfalls neu,

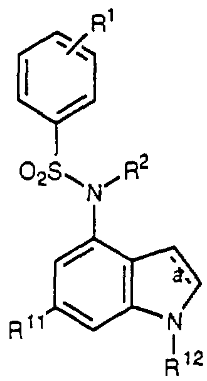

Ie

worin $R^1$, $R^2$, $R^{11}$, $R^{12}$ und a die in Formel I angegebene Bedeutung haben.

Die Herstellung dieser neuen Verbindungen wird im Beispiel 52 beschrieben.

Die Verbindungen der Formel I und ihre Salze, soweit sie nicht bekannt und ihre Herstellung bereits beschrieben ist, können in an sich bekannter Weise aus einer Verbindung der Formel II hergestellt werden,

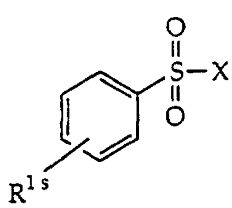

II

worin

$R^{1s}$ die für $R^1$ angegebene Bedeutung hat oder geschütztes Amino und

X Halogen oder -NHY bedeuten, und Y für ein Alkalimetall, beispielsweise Natrium oder Kalium, steht,

und zwar dadurch, dass man eine Verbindung der Formel II, in der X Halogen bedeutet,

a) mit einer Verbindung der Formel

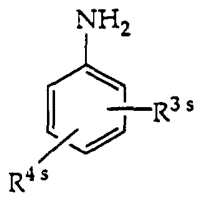

III a

worin

$R^{3s}$ Wasserstoff, $C_{1-7}$-Alkoxy, Halogen, geschütztes Amino oder geschütztes $C_{1-7}$-Alkylamino und
$R^{4s}$ geschütztes Amino oder geschütztes $C_{1-7}$-Alkylaminobedeuten, umsetzt und die Aminoschutzgruppen abspaltet; oder

b) mit einer Verbindung der Formel

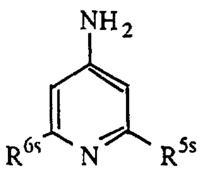

III b

worin

$R^{5s}$ Halogen und
$R^{6s}$ Halogen, $C_{1-7}$-Alkyl oder $CF_3$ bedeuten, umsetzt und das Reaktionsprodukt gegebenenfalls mit $C_{1-7}$-Alkylamin oder di- $C_{1-7}$-Alkylamin behandelt und die Aminoschutzgruppe abspaltet ; oder

c) mit einer Verbindung der Formel

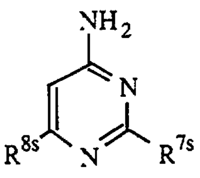

III c

worin

$R^{7s}$ Mercapto und
$R^{8s}$ Amino, $C_{1-7}$-Alkylamino, di- $C_{1-7}$-Alkylamino, Benzylamino, $C_{1-7}$-Alkoxy, Pyrrolidin-1-yl oder Azetidin-1-yl bedeuten, umsetzt und gegebenenfalls die Aminoschutzgruppe abspaltet, oder

d) eine Verbindung der Formel II, in der X -NHY bedeutet, zunächst mit einer Verbindung der Formel

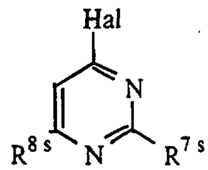

IIId

worin

$R^{7s}$ Amino, $C_{1-7}$-Alkylamino, di- $C_{1-7}$-Alkylamino, Pyrrolidin-1-yl oder Azetidin-1-yl und
$R^{8s}$ Halogen bedeuten, umsetzt, und dann das Reaktionsprodukt gegebenenfalls mit einem $C_{1-7}$-Alkylamin, di- $C_{1-7}$-Alkylamin,Azetidin, Pyrrolidin oder einem Alkoholat behandelt; oder

e) eine Verbindung der Formel II, in der X Halogen bedeutet, mit einerVerbindung der Formel

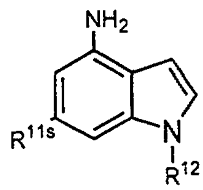

IIIe

worin
$R^{11s}$ Wasserstoff oder Halogen und $R^{12}$ Wasserstoff oder $C_{1-7}$-Alkyl bedeuten, umsetzt und anschliessend gegebenenfalls zu eine Verbindung der Formel Ie reduziert, worin a keine Doppelbindung bedeutet, oder dadurch, dass man
f) eine Verbindung der Formel

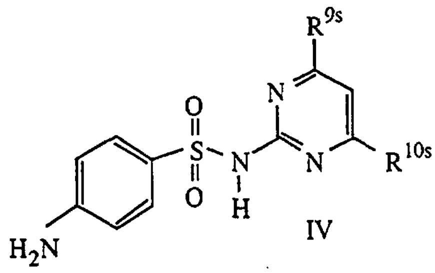

IV

worin
$R^{9s}$ und $R^{10s}$ $C_{1-7}$-Alkoxy bedeuten, mit einem niederen Alkylamin umsetzt, oder
g) ein Sulfadimethoxin der Formel V

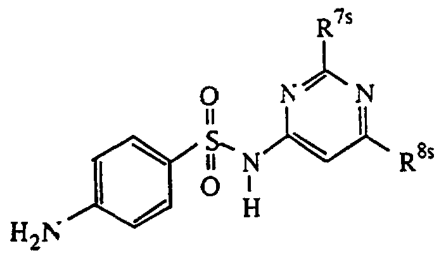

V

worin

$R^{7s}$ und $R^{8s}$ $C_{1-7}$-Alkoxy bedeuten, mit einem $C_{1-7}$-Alkylamin umsetzt und

h) erwünschtenfalls eine Verbindung der allgemeinen Formel I in ein pharmazeutisch annehmbares Salz überführt.

Alle hier erwähnten Verfahrensvarianten können in an sich bekannter Weise durchgeführt werden.

Zur Herstellung von Verbindungen der Formel Ia werden die Ausgangsverbindungen der Formeln II und IIIa zweckmässigerweise bei Raumtemperatur in einem geeigneten Lösungsmittel, beispielsweise Pyridin, umgesetzt.

Zur Alkylierung der Aminogruppe kann die geschützte Aminogruppe beispielsweise mit niederem Alkyljodid umgesetzt werden.

Eine geeignete Aminoschutzgruppe ist die Acetyl-Gruppe.

Die Abspaltung der Aminoschutzgruppe erfolgt durch Zugabe einer Base ( z. B. NaOH) durch Erhitzen unter Rückfluss.

Bei Verbindungen der Formel Ia ist die Schutzgruppenabspaltung jeweils der letzte Reaktionsschritt.

Zur Herstellung von Verbindungen der Formeln Ib werden die Ausgangsverbindungen der Formeln II (X = Halogen) und IIIb zweckmässigerweise bei 20-80 °C, bevorzugt bei 60 °C in einem geeigneten Lösungsmittel, beispielsweise Pyridin, umgesetzt.

Zur Herstellung von Verbindungen der Formel Ic werden die Ausgangsverbindungen der Formeln II (X = Halogen), mit Verbindungen der Formel IIIc umgesetzt.

Die Abspaltung der Aminoschutzgruppe erfolgt durch Basen-behandlung wie oben beschrieben.

Das Reaktionsprodukt der Umsetzung-einer Verbindung der Formel II mit einer Verbindung der Formel IIIb, beispielsweise das 4-Amino-N-(2,6-dibrom-pyridin-4-yl)-benzolsulfonamid, kann zur Herstellung von Verbindungen der Formel Ib, worin $R^5$ Halogen, $C_{1-7}$-Alkylamino oder di- $C_{1-7}$-Alkylamino und $R^6$ $C_{1-7}$-Alkylamino oder di- $C_{1-7}$-Alkylamino bedeuten, durch Umsetzung mit einem $C_{1-7}$-Alkylamin oder di- $C_{1-7}$-Alkylamin bei 60-200 °C, verwendet werden.

Zur Herstellung von Verbindungen der Formel I, worin $R^2$ $C_{1-7}$-Alkyl bedeutet, kann ein entsprechendes Sulfonamid mit Diazomethan in an sich bekannter Weise umgesetzt werden ( Beispiel 19).

Die für die Herstellung der Verbindungen der Formel I benötigten Ausgangsstoffe sind bekannte Verbindungen oder können in Analogie zu bekannten Verfahren hergestellt werden. Diese Umsetzungen sind jedem Fachmann geläufig.

Die Bindung der erfindungsgemässen Verbindungen der Formel I an 5-$HT_6$ Receptoren wurde wie folgt bestimmt.

Es wurden Membranen verwendet, die von HEK 293 Zellen erhalten wurden, die mit 5-$HT_6$ Rezeptoren von Ratten transfektiert waren.

Die Zellen wurden durch zweimaliges Zentrifugieren ( 10 Minuten bei 3000 g) in Phosphatpuffer-Kochsalzlösung gereinigt. Die Zellmasse wurde in einer eiskalten Lösung bestehend aus 50 mM Tris-HCl-Puffer, 10 mM $MgCl_2$, 0.5 mM EDTA und 0.1 mM Phenylmethylsulphonylfluorid suspendiert und homogenisiert. ( Polytron Homogenisierer, 15 Sekunden bei maximaler Geschwindigkeit. Das Homogenisat wurde 10 Minuten bei 37 °C inkubiert und anschliessend zentrifugiert ( 20 Minuten bei 20 000 g). Die Zellmasse wurde nochmals in der oben genannten Tris-Puffer-Lösung suspendiert. Die erhaltene Zellkonzentration betrug 4 x $10^7$ Zellen/ml. Aliquote Anteile des Homogenisats von je 1 ml wurden bei (-80) °C eingefroren.

Zur Bestimmung der Affinität der Testsubstanz zum 5-$HT_6$ Rezeptor wurden Verdrängungsversuche durchgeführt. Zur Durchführung des Tests wurde das Homogenisat aufgetaut und in einer Puffer-Lösung ( pH 7.4) bestehend aus 50 mM Tris-HCl-Puffer, 5 mM $MgCl_2$, $10^{-5}$ M Pargylin und 0.1% Ascorbinsäure suspendiert. 100 µl Membransuspension, 50 µl [$^3$H]-LSD ( spezifische Aktivität 85 Ci/mMol, Endkonzentration 1 nM) und 50 µl Testsubstanzlösung wurden 1 Stunde bei 37 °C inkubiert. Die jeweilige Testsubstanz wurde bei 7 verschiedenen Konzentrationen von $10^{-10}$ M bis $10^{-4}$ M untersucht. Die Bindungsreaktion der Testsubstanz wurde durch schnelle Filtration durch Whatmann GF/B-Filter unterbrochen. Die Filter wurden mit 2 x 2 ml Tris-HCl Puffer (50 mM, pH 7.4) gewaschen und die Radioaktivität

der Filter wurde durch Szintillationsspektroskopie in 2 ml Szintillationslösung gemessen. Alle Tests wurden dreifach durchgeführt und drei Mal wiederholt.

Es sind die pKi-Werte (pKi = $-\log_{10}$Ki) der zu prüfenden Substanzen bestimmt worden. Der Ki-Wert ist durch folgende Formel definiert:

$$Ki = \frac{IC_{50}}{1 + \frac{[L]}{K_D}}$$

wobei die $IC_{50}$ - Werte diejenigen Konzentrationen der zu prüfenden Verbindungen in nM sind, durch welche 50% des am Rezeptor gebundenen Liganden verdrängt werden. [L] ist die Konzentration des Liganden und der $K_D$-Wert die Dissoziationskonstante des Liganden.

Die erfindungsgemässen Verbindungen besitzen eine selektive Affinität zu 5-HT 6 Rezeptoren mit einem Ki-Wert unter 1.6 μM.

Die Verbindungen der Formel I und die pharmazeutisch annehmbaren Salze der Verbindungen der Formel I können als Heilmittel, z.B. in Form von pharmazeutischen Präparaten, Verwendung finden. Die pharmazeutischen Präparate können oral, z.B. in Form von Tabletten, Lacktabletten, Dragées, Hart- und Weichgelatinekapseln, Lösungen, Emulsionen oder Suspension, verabreicht werden. Die Verabreichung kann aber auch rektal, z.B. in Form von Suppositorien, parenteral, z.B. in Form von Injektionslösungen erfolgen.

Zur Herstellung von pharmazeutischen Präparaten können die Verbindungen der Formel I und die pharmazeutisch annehmbaren Salze der Verbindungen der Formel I mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Oele, Wachse, Fette, halbfeste und flüssige Polyole und dergleichen. Je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Glyzerin, pflanzliches Oel und dergleichen. Für Suppositorien eignen sich als Träger beispielsweise natürliche oder gehärtete Oele, Wachse, Fette, halbflüssige oder flüssige Polyole und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Arzneimittel, enthaltend eine Verbindung der Formel I oder ein pharmazeutisch annehmbares Salz davon und einen therapeutisch inerten Träger, sind ebenfalls Gegenstand der vorliegenden Erfindung, ebenso ein Verfahren zu deren Herstellung, das dadurch gekennzeichnet ist, dass man eine oder mehrere Verbindungen der Formel I und/oder pharmazeutisch annehmbare Säureadditionssalze wertvolle Stoffe zusammen mit einem oder mehreren therapeutisch inerten Trägern in eine galenische Darreichungsform bringt.

Erfindungsgemäss kann man Verbindungen der allgemeinen Formel I sowie deren pharmazeutisch annehmbare Salze bei der Behandlung oder Verhütung von zentralnervösen Störungen, wie Depressionen, Psychosen, Schizophrenie, neurologische Störungen, Gedächtnisstörungen, Parkinsonsche Krankheit, amyotrophe Lateralsklerose, Alzheimer Krankheit, Huntingtonsche Krankheit und zur Herstellung entsprechender Arzneimittel verwenden. Die Dosierung kann innerhalb weiter Grenzen variieren und ist natürlich in jedem einzelnen Fall den individuellen Gegebenheiten anzupassen. Bei oraler Verabreichung liegt die Dosis in einem Bereich von etwa 0,01 mg pro Dosis bis etwa 1000 mg pro Tag einer Verbindung der allgemeinen Formel I bzw. der entsprechenden Menge eines pharmazeutisch annehmbaren Salzes davon, wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken.

### Beispiel 1

<u>4-Amino-N-(2-amino-6-methylamino-pvrimidin-4-yl)-benzolsulfonamid</u>

0.98 g (0.006 Mol) 2-Amino-4,6-dichlor-pyrimidin und 3.03 g (0.012 Mol) N-(4-Sulfamoyl-phenyl)-acetamid Kaliumsalz wurden 8 Std. in 10 ml 1-Methyl-2-pyrrolidon bei 140°C gerührt. Dann destillierte man das Lösungsmittel am Hochvakuum ab, verteilte den Rückstand auf Essigester/Wasser, sättigte die anorganische Phase mit Kochsalz und destillierte den restlichen in der Wasserphase gelösten Essigester am Rotationsverdampfer ab. Die Wasserphase

wurde mit 1N HCl sauer gestellt, die ausgefallenen Kristalle abgenutscht und mit Wasser gewaschen. Nach Trocknen erhielt man 1.37 g (67%) N-[4-(2-Amino-6-chlor-pyrimidin-4-ylsulfamoyl)-phenyl]-acetamid als beige Kristalle; Smp.: 272-273°C (Zers.).

0.885 g (0.0026 Mol) N-[4-(2-Amino-6-chlor-pyrimidin-4-ylsulfamoyl)-phenyl]-acetamid wurden in 31 ml 0.5N NaOH gelöst und 3 Std. am Rückfluss gekocht. Man extrahierte mit Essigester, sättigte die wässrige Phase mit Kochsalz und destillierte den restlichen Essigester am Rotationsverdampfer ab. Dann wurde die wässrige Phase mit 3N HCl sauer gestellt und der ausgefallene Niederschlag abgenutscht. Nach Trocknen erhielt man 0.76 g (86%) 4-Amino-N-(2-amino-6-chlor-pyrimidin-4-yl)-benzolsulfonamid als beige Kristalle; Smp.: >265°C (Zers.).

0.527 g (0.00176 Mol) 4-Amino-N-(2-amino-6-chlor-pyrimidin-4-yl)-benzolsulfonamid wurden in 22 ml (0.176 Mol) 8 M Methylamin in Ethanol gelöst und in einem Autoklaven 16 Std. bei 130°C gerührt. Die erhaltene Suspension wurde filtriert, der Niederschlag in Methanol gelöst und mit Aktivkohle behandelt, filtriert und vom Lösungsmittel befreit. Der Rückstand wurde in Ethanol suspendiert und filtriert. Man erhielt 0.055 g (10%) 4-Amino-N-(2-amino-6-methylamino-pyrimidin-4-yl)-benzolsulfonamid als beige Kristalle; Smp.: 285°C (Zers.).

**Beispiel 2**

4-Amino-N-(2-amino-6-ethylamino-pyrimidin-4-yl)-benzolsulfonamid

0.50 g (0.00167 Mol) 4-Amino-N-(2-amino-6-chlor-pyrimidin-4-yl)-benzolsulfonamid und 11 ml (0.167 Mol) Ethylamin wurden in 20 ml Ethanol gelöst und in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.271 g (53%) 4-Amino-N-(2-amino-6-ethylamino-pyrimidin-4-yl)-benzolsulfonamid als beige Kristalle; Smp.: 272-274°C.

**Beispiel 3**

4-Amino-N-(2-amino-6-benzylamino-pyrimidin-4-yl)-benzolsulfonamid

0.30 g (0.001 Mol) 4-Amino-N-(2-amino-6-chlor-pyrimidin-4-yl)-benzolsulfonamid und 11 ml (0.1 Mol) Benzylamin wurden in 15 ml Ethanol gelöst und in einem Autoklaven 3 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.15 g (41%) 4-Amino-N-(2-amino-6-benzylamino-pyrimidin-4-yl)-benzolsulfonamid als beige Kristalle; Smp.: 221°C (Zers.).

**Beispiel 4**

4-Amino-N-(2,6-bis-methylamino-pyrimidin-4-yl)-benzolsulfonamid

5.0 g (0.016 Mol) Sulfadimethoxin wurden in 60 ml 33 prozentigem ethanolischem Methylamin gelöst und für 30 Std. bei 150°C in einem Autoklav gerührt. Man kühlte ab, befreite vollständig von den Lösungsmitteln, verrührte 2 Std. in 70 ml Methanol und nutschte ab. Man erhielt 4.2 g (84%) 4-Amino-N-(2,6-bis-methylamino-pyrimidin-4-yl)-benzolsulfonamid als graue Kristalle; Smp. 303-305°C.

11.5 g (0.0373 Mol) 4-Amino-N-(2,6-bis-methylamino-pyrimidin-4-yl)-benzolsulfonamid wurden mit 11.5 ml 25 prozentiger wässriger Salzsäure versetzt und 1 Std. bei 0°C nachgerührt. Das Wasser wurde vollständig eingedampft und der Rückstand wurde aus Ethanol/Diethylether umkristallisiert. Man erhielt 11.8 g (89%) 4-Amino-N-(2,6-bis-methylaminopyrimidin-4-yl)-benzolsulfonamid Hydrochlorid (1:1.8) als hellgelbe Kristalle; Smp. 175-250°C (Zers.).

**Beispiel 5**

4-Amino-N-(6-ethylamino-2-methylamino-pyrimidin-4-yl)-benzolsulfonamid

2.0 g (0.011 Mol) (4,6-Dichlorpyrimidin-2-yl)-methylamin und 5.67 g (0.022 Mol) N-(4-Sulfamoyl-phenyl)-acetamid Kaliumsalz wurden 8 Std. in 10 ml 1-Methyl-2-pyrrolidon bei 140°C gerührt. Dann destillierte man das Lösungsmittel am Hochvakuum ab, verteilte den Rückstand auf Essigester/Wasser, sättigte die anorganische Phase mit Kochsalz und destillierte den restlichen in der Wasserphase gelösten Essigester am Rotationsverdampfer ab. Die Wasserphase wurde mit 1N HCl sauer gestellt, die ausgefallenen Kristalle abgenutscht und mit Wasser gewaschen. Nach Trocknen erhielt man 2.72 g (68%) N-[4-(6-Chlor-2-methylamino-pyrimidin-4-ylsulfamoyl)-phenyl]-acetamid als beige Kristalle; Smp.: > 240°C (Zers.).

2.7 g (0.008 Mol) N-[4-(6-Chlor-2-methylamino-pyrimidin-4-ylsulfamoyl)-phenyl]-acetamid wurden in 76 ml 1N NaOH gelöst und 3 Std. am Rückfluss gekocht. Man extrahierte mit Essigester, sättigte die wässrige Phase mit Kochsalz und destillierte den restlichen Essigester am Rotationsverdampfer ab. Dann wurde die wässrige Phase mit 3N HCl sauer gestellt und der ausgefallene Niederschlag abgenutscht. Nach Trocknen erhielt man 2.12 g (89%) 4-Amino-N-(6-chlor-2-methylamino-pyrimidin-4-yl)-benzolsulfonamid Hydrochlorid(1:1.9) als weisse Kristalle; MS (ISN): me/e = 312 ($C_{11}H_{11}ClN_5O_2S^-$).

0.314 g (0.001 Mol) 4-Amino-N-(6-chlor-2-methylamino-pyrimidin-4-yl)-benzolsulfonamid und 6.6 ml (0.1 Mol) Ethylamin wurden in 15 ml Ethanol in einem Autoklaven 3 Std. bei 130°C gerührt. Das Reaktionsgemisch wurde vom Lösungsmittel befreit, der Rückstand in 8 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.18 g (56%) 4-Amino-N-(6-ethylamino-2-methylamino-pyrimidin-4-yl)-benzolsulfonamid als weisse Kristalle; Smp.: 252°C (Zers.).

**Beispiel 6**

<u>4-Amino-N-(6-isopropylamino-2-methylamino-pyrimidin-4-yl)-benzolsulfonamid</u>

0.314 g (0.001 Mol) 4-Amino-N-(6-chlor-2-methylamino-pyrimidin-4-yl)-benzolsulfonamid und 6.6 ml (0.1 Mol) Ethylamin wurden in 15 ml Ethanol in einem Autoklaven 3 Std. bei 130°C gerührt. Das Reaktionsgemisch wurde vom Lösungsmittel befreit, der Rückstand in 8 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.12 g (36%) 4-Amino-N-(6-isopropylamino-2-methylamino-pyrimidin-4-yl)-benzolsulfonamid als beige Kristalle; Smp.: 240°C (Zers.).

**Beispiel 7**

<u>4-Amino-N-(6-dimethylamino-2-methylamino-pyrimidin-4-yl)-benzolsulfonamid</u>

0.314 g (0.001 Mol) 4-Amino-N-(6-chlor-2-methylamino-pyrimidin-4-yl)-benzolsulfonamid und 18 ml (0.1 Mol) Dimethylamin in Ethanol (5.6 M) wurden in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.30 g (78%) 4-Amino-N-(6-dimethylamino-2-methylamino-pyrimidin-4-yl)-benzolsulfonamid als beige Kristalle; Smp.: > 300°C.

**Beispiel 8**

<u>4-Amino-N-(6-azetidin-1-yl-2-methylamino-pyrimidin-4-yl)-benzolsulfonamid</u>

0.314 g (0.001 Mol) 4-Amino-N-(6-chlor-2-methylamino-pyrimidin-4-yl)-benzolsulfonamid und 1.0 ml (0.015 Mol) Trimethylenimin wurden in 20 ml Ethanol in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.24 g (72%) 4-Amino-N-(6-azetidin-1-yl-2-methylamino-pyrimidin-4-yl)-benzolsulfonamid als weisse Kristalle; Smp.: 295-296°C.

**Beispiel 9**

<u>4-Amino-N-(2-methylamino-6-pyrrolidin-1-yl-pyrimidin-4-yl)-benzolsulfonamid</u>

0.314 g (0.001 Mol) 4-Amino-N-(6-chlor-2-methylamino-pyrimidin-4-yl)-benzolsulfonamid und 2.5 ml (0.015 Mol) Pyrrolidin wurden in 20 ml Ethanol in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 150 ml 1N NaOH gelöst und filtriert. Das Filtrat stellte man mit 1N HCl auf pH = 6. Der Niederschlag wurde abgenutscht, mit Wasser gewaschen und getrocknet. Man erhielt 0.22 g (63%) 4-Amino-N-(2-methylamino-6-pyrrolidin-1-yl-pyrimidin-4-yl)-benzolsulfonamid als beige Kristalle; Smp.: > 300°C.

**Beispiel 10**

4-Amino-N-(2-ethylamino-6-methylamino-pyrimidin-4-yl)-benzolsulfonamid

2.0 g (0.011 Mol) (4,6-Dichlorpyrimidin-2-yl)-ethylamin und 5.67 g (0.022 Mol) N-(4-Sulfamoyl-phenyl)-acetamid Kaliumsalz wurden 8 Std. in 10 ml 1-Methyl-2-pyrrolidon bei 140°C gerührt. Dann destillierte man das Lösungsmittel am Hochvakuum ab, verteilte den Rückstand auf Essigester/Wasser, sättigte die anorganische Phase mit Kochsalz und destillierte den restlichen in der Wasserphase gelösten Essigester am Rotationsverdampfer ab. Die Wasserphase wurde mit 1N HCl sauer gestellt, die ausgefallenen Kristalle abgenutscht und mit Wasser gewaschen. Nach Trocknen erhielt man 2.88 g (75%) N-[4-(6-Chlor-2-ethylamino-pyrimidin-4-ylsulfamoyl)-phenyl]-acetamid als beige Kristalle, die so direkt in der nächsten Stufe eingesetzt wurden.

2.88 g (0.0075 Mol) N-[4-(6-Chlor-2-ethylamino-pyrimidin-4-ylsulfamoyl)-phenyl]-acetamid wurden in 76 ml 1N NaOH gelöst und 3 Std. am Rückfluss gekocht. Man extrahierte mit Essigester, sättigte die wässrige Phase mit Kochsalz und destillierte den restlichen Essigester am Rotationsverdampfer ab. Dann wurde die wässrige Phase mit 3N HCl sauer gestellt und der ausgefallene Niederschlag abgenutscht. Nach Trocknen erhielt man 2.2 g (90%) 4-Amino-N-(6-chlor-2-ethylamino-pyrimidin-4-yl)-benzolsulfonamid als weisse Kristalle; Smp.: 172-173°C.

0.1 g (0.00031 Mol) 4-Amino-N-(6-chlor-2-ethylamino-pyrimidin-4-yl)-benzolsulfonamid wurden in 20 ml Ethanol gelöst und mit 0.6 ml (0.0043 Mol) Trimethylamin und 0.1 g (0.0015 Mol) Methylamin Hydrochlorid in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 150 ml 1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.053 g (54%) 4-Amino-N-(2-ethylamino-6-methylamino-pyrimidin-4-yl)-benzolsulfonamid als beige Kristalle; Smp.: 261-263°C.

**Beispiel 11**

4-Amino-N-(2,6-bis-ethylamino-pvrimidin-4-yl)-benzolsulfonamid

1.50 g (0.00483 Mol) Sulfadimethoxin wurden in 25 ml Ethanol gelöst, mit 12.5 ml ( 0.193 Mol) kühlem Ethylamin versetzt und für 24 Std. bei 180°C in einem Autoklav gerührt. Man kühlte ab und befreite vollständig von den Lösungsmitteln. Der Rückstand wurde in 150 ml Ethanol heiss suspendiert und abgenutscht. Man erhielt 0.79 g (48%) 4-Amino-N-(2,6-bis-ethylaminopyrimidin-4-yl)-benzolsulfonamid als hellbeige Kristalle; Smp. 245-250°.

0.79 g (0.00234 Mol) 4-Amino-N-(2,6-bis-ethylamino-pyrimidin-4-yl)-benzolsulfonamid wurden in 150 ml Methanol gelöst, mit 2.0 ml (0.0070 Mol) 3.5 N ethanolischer Salzsäure versetzt und 2 Std. bei 0°C nachgerührt. Die Lösung wurde vollständig von den Lösungsmitteln befreit und der Rückstand wurde aus Ethanol/Diethylether umkristallisiert. Man erhielt 0.61 g (70%) 4-Amino-N-(2,6-bis-ethylamino-pyrimidin-4-yl)-benzolsulfonamid Hydrochlorid als hellbeige Kristalle; Smp. 197-208°C.

**Beispiel 12**

4-Amino-N-(2-ethylamino-6-isopropylamino-pyrimidin-4-yl)-benzolsulfonamid

0.5 g (0.0015 Mol) 4-Amino-N-(6-chlor-2-ethylamino-pyrimidin-4-yl)-benzolsulfonamid wurden in 20 ml Ethanol gelöst und mit 13 ml (0.15 Mol) Isopropylamin in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde abfiltriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.20 g (37%) 4-Amino-N-(2-ethylamino-6-isopropylamino-pyrimidin-4-yl)-benzolsulfonamid als weisse Kristalle; Smp.: 258-259°C.

**Beispiel 13**

4-Amino-N-(6-dimethylamino-2-ethylamino-pyrimidin-4-yl)-benzolsulfonamid

0.5 g (0.0015 Mol) 4-Amino-N-(6-chlor-2-ethylamino-pyrimidin-4-yl)-benzolsulfonamid wurden in 20 ml 33%ige Dimethylamin in Ethanol gelöst und in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt.

Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.426 g (83%) 4-Amino-N-(6-dimethylamino-2-ethylamino-pyrimidin-4-yl)-benzolsulfonamid als weisse Kristalle; Smp.: 301-302°C.

**Beispiel 14**

4-Amino-N-(6-azetidin-1-yl-2-ethylamino-pyrimidin-4-yl)-benzolsulfonamid

0.3 g (0.000915 Mol) 4-Amino-N-(6-chlor-2-ethylamino-pyrimidin-4-yl)-benzolsulfonamid wurden in 20 ml Ethanol gelöst, mit 0.93 ml (0.0137 Mol) Trimethylenimin versetzt und in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.248 g (78%) 4-Amino-N-(6-azetidin-1-yl-2-ethylamino-pyrimidin-4-yl)-benzolsulfonamid als weisse Kristalle; Smp.: 292-293°C.

**Beispiel 15**

4-Amino-N-(2,6-bis-propylamino-pyrimidin-4-yl)-benzolsulfonamid

1.50 g (0.00483 Mol) Sulfadimethoxin wurden in 20 ml Ethanol suspendiert, mit 16 ml ( 0.193 Mol) Propylamin versetzt und für 65 Std. bei 140°C in einem Autoklav gerührt. Man kühlte ab und nutschte die nach 24 Std. ausgefallenen Kristalle ab. Man erhielt 0.75 g (50%) 4-Amino-N-(2,6-bis-propylamino-pyrimidin-4-yl)-benzolsulfonamid als hellbeige Kristalle; Smp. 211-215°C.

0.75 g (0.00205 Mol) 4-Amino-N-(2,6-bis-propylamino-pyrimidin-4-yl)-benzolsulfonamid wurden in 100 ml Methanol gelöst, mit 1.75 ml (0.0062 Mol) 3.5 N ethanolischer Salzsäure versetzt und 2 Std. bei 0°C nachgerührt. Die Lösung wurde vollständig von den Lösungsmitteln befreit und der Rückstand wurde aus Ethanol/Diethylether umkristallisiert. Man erhielt 0.68 g (82%) 4-Amino-N-(2,6-bis-propylamino-pyrimidin-4-yl)-benzolsulfonamid Hydrochlorid als beige Kristalle; Smp. 189-195°C.

**Beispiel 16**

4-Amino-N-(6-ethylamino-2-isopropylamino-pyrimidin-4-yl)-benzolsulfonamid

2.42 g (0.0117 Mol) (4,6-Dichlorpyrimidin-2-yl)-ethylamin und 5.0 g (0.020 Mol) N-(4-Sulfamoyl-phenyl)-acetamid Kaliumsalz wurden 8 Std. in 10 ml 1-Methyl-2-pyrrolidon bei 140°C gerührt. Dann destillierte man das Lösungsmittel am Hochvakuum ab, verteilte den Rückstand auf Essigester/Wasser, sättigte die anorganische Phase mit Kochsalz und destillierte den restlichen in der Wasserphase gelösten Essigester am Rotationsverdampfer ab. Die Wasserphase wurde mit 1N HCl sauer gestellt, der dabei ausgefallene Niederschlag abgenutscht und mit Wasser gewaschen. Dieser wurde ohne Trocknung so direkt in der nächsten Stufe eingesetzt.

Das in der vorher beschriebenen Stufe erhaltene Rohprodukt wurde in 100 ml 1N NaOH gelöst und 3 Std. am Rückfluss gekocht. Man extrahierte mit Essigester, sättigte die wässrige Phase mit Kochsalz und destillierte den restlichen Essigester am Rotationsverdampfer ab. Dann wurde die wässrige Phase mit 3N HCl sauer gestellt und der dabei ausgefallene Niederschlag abgenutscht. Nach Trocknen erhielt man 2.22 g (55%, bezogen auf in der vorherigen Stufe eingesetztes (4,6-Dichlorpyrimidin-2-yl)-ethylamin) 4-Amino-N-(6-chlor-2-isopropylamino-pyrimidin-4-yl)-benzolsulfonamid als weisse Kristalle; Smp.: 94-95°C.

0.1 g (0.000293 Mol) 4-Amino-N-(6-chlor-2-isopropylamino-pyrimidin-4-yl)-benzolsulfonamid wurden in 20 ml Ethanol gelöst, mit 1.93 ml (0.0293 Mol) Ethylamin versetzt und in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde abfiltriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.044 g (43%) 4-Amino-N-(6-ethylamino-2-isopropylamino-pyrimidin-4-yl)-benzolsulfonamid als weisse Kristalle; Smp.: 266-267°C.

**Beispiel 17**

4-Amino-N-(2-dimethylamino-6-methylamino-pyrimidin-4-yl)-benzolsulfonamid

0.50 g (0.00153 Mol) 4-Amino-N-(2-dimethylamino-6-chlor-pyrimidin-4-yl)-benzolsulfonamid wurden in 20 ml (0.176 Mol) 8 M Methylamin in Ethanol gelöst und in einem Autoklaven 4 Std. bei 130°C gerührt. Das Reaktionsgemisch wurde vom Lösungsmittel befreit und der Rückstand auf Kieselgel mit Methanol/Dichlormethan 1:19 chromatographiert. Man erhielt 0.10 g (21%) 4-Amino-N-(2-dimethylamino-6-methylamino-pyrimidin-4-yl)-benzolsulfonamid als beige Kristalle; Smp.: 253-254°C.

**Beispiel 18**

4-Amino-N-(2-dimethylamino-6-ethylamino-pyrimidin-4-yl)-benzolsulfonamid

0.2 g (0.00061 Mol) 4-Amino-N-(6-chlor-2-dimethylamino-pyrimidin-4-yl)-benzolsulfonamid wurden in 20 ml Ethanol gelöst, mit 4.2 ml (0.061 Mol) Ethylamin versetzt und in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.082 g (40%) 4-Amino-N-(2-dimethylamino-6-ethylamino-pyrimidin-4-yl)-benzolsulfonamid als beige Kristalle; Smp.: 237-238°C.

**Beispiel 19**

4-Amino-N-(2,6-bis-methylamino-pyrimidin-4-yl)-N-methyl-benzolsulfonamid

0.65 g (0.0021 Mol) 4-Amino-N-(2,6-bis-methylamino-pyrimidin-4-yl)-benzolsulfonamid wurden in einem Gemisch aus 100 ml Methanol und 400 ml Dimethylformamid gelöst und mit 60 ml einer Lösung von Diazomethan in Diethylether versetzt. Man rührte 30 Min. bei Raumtemperatur, destillierte das Lösungsmittel ab und chromatographierte den Rückstand über 50 g $SiO_2$ mit 5% Methanol in Methylenchlorid als Laufmittel. Man erhielt 0.24 g (35%) 4-Amino-N-(2,6-bis-methylamino-pyrimidin-4-yl)-N-methyl-benzolsulfonamid als gelben Feststoff; Smp.: 79-80°C.

**Beispiel 20**

4-Amino-N-(6-azetidin-1-yl-2-dimethylamino-pyrimidin-4-yl)-benzolsulfonamid

0.20 g (0.00061 Mol) 4-Amino-N-(6-chlor-2-dimethylamino-pyrimidin-4-yl)-benzolsulfonamid wurden in 20 ml Ethanol gelöst, mit 0.62 ml (0.0092 Mol) Trimethylenimin versetzt und in einem Autoklaven 4 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde abfiltriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.13 g (61%) 4-Amino-N-(6-azetidin-1-yl-2-dimethylaminopyrimidin-4-yl)-benzolsulfonamid als beige Kristalle; Smp.: 239-240°C.

**Beispiel 21**

4-Amino-N-(2-azetidin-1-yl-6-methylamino-pyrimidin-4-yl)-benzolsulfonamid

2.0 ml (0.030 Mol) Trimethylenimin und 2.30 ml (0.020 Mol) 2,4,6-Trichlorpyrimidin wurden in 50 ml Ethanol 3 Std. bei Raumtemperatur gerührt. Man destillierte das Lösungsmittel ab, suspendierte den Rückstand in Diethylether und wusch mit gesättigter $NaHCO_3$-Lösung. Die organische Phase wurde über $MgSO_4$ getrocknet, eingeengt und der Rückstand auf Kieselgel mit Diethylether/Hexan 1:5 bis 2:1 (Gradientenelution) chromatographiert. Die erste Fraktion ($R_f$. 0.39, Diethylether/Hexan 1:3) enthielt 0.67 g (16%) 2-Azetidin-1-yl-4,6-dichlor-pyrimidin als weisse Kristalle; Smp.: 109°C. Die zweite Fraktion enthielt 1.50 g (37%) 2,4-Dichlor-6-azetidin-1-yl-pyrimidin als weisse Kristalle; Smp.: 129-130°C.

0.65 g (0.0032 Mol) 2-Azetidin-1-yl-4,6-dichlor-pyrimidin und 1.6 g (0.0064 Mol) N-(4-Sulfamoyl-phenyl)-acetamid Kaliumsalz wurden 8 Std. in 10 ml 1-Methyl-2-pyrrolidon bei 140°C gerührt. Dann destillierte man das Lösungsmittel am Hochvakuum ab, verteilte den Rückstand auf Essigester/Wasser, sättigte die anorganische Phase mit Kochsalz und destillierte den restlichen in der Wasserphase gelösten Essigester am Rotationsverdampfer ab. Die Wasserphase wurde mit 1N HCl sauer gestellt und der ausgefallene Niederschlag abgenutscht und mit Wasser gewaschen. Dieser wurde ohne Trocknung so direkt in der nächsten Stufe eingesetzt.

Das in der vorher beschriebenen Stufe erhaltene Rohprodukt wurde in 50 ml 1N NaOH gelöst und 2 Std. am Rückfluss gekocht. Man extrahierte mit Essigester, sättigte die wässrige Phase mit Kochsalz und destillierte den restlichen Essigester am Rotationsverdampfer ab. Dann wurde die wässrige Phase mit 3N HCl sauer gestellt und der dabei ausgefallene Niederschlag abgenutscht. Nach Trocknen erhielt man 0.66 g (60%, bezogen auf in der vorherigen Stufe eingesetztes 2-Azetidin-1-yl-4,6-dichlor-pyrimidin) 4-Amino-N-(2-azetidin-1-yl-6-chlor-pyrimidin-4-yl)-benzolsulfonamid als beige Kristalle; Smp.: > 203°C (Zers.).

0.20 g (0.00059 Mol) 4-Amino-N-(2-azetidin-1-yl-6-chlor-pyrimidin-4-yl)-benzolsulfonamid wurden in 20 ml Ethanol gelöst, mit 0.4 g (0.00588 Mol) Methylamin Hydrochlorid und 2.28 ml (0.0176 Mol) Triethylamin versetzt und 8 Std. bei 130°C im Autoklaven gerührt. Das Reaktionsgemisch wurde vom Lösungsmittel befreit und der Rückstand auf Kieselgel mit Methanol/Dichlormethan 1:19 chromatographiert. Man erhielt 0.053 g (27%) 4-Amino-N-(2-azetidin-1-yl-6-methylamino-pyrimidin-4-yl)-benzolsulfonamid als leicht beige Kristalle; Smp.: > 260°C (Zers.).

**Beispiel 22**

4-Amino-N-(2-azetidin-1-yl-6-ethylamino-pyrimidin-4-yl)-benzolsulfonamid

0.2 g (0.00061 Mol) 4-Amino-N-(2-azetidin-1-yl-6-chlor-pyrimidin-4-yl)-benzolsulfonamid wurden in 20 ml Ethanol gelöst, mit 3.9 ml (0.061 Mol) Ethylamin versetzt und in einem Autoklaven 12 Std. bei 130°C gerührt. Das Reaktionsgemisch wurde vom Lösungsmittel befreit, der Rückstand in 5 ml Ethanol suspendiert und 15 Min. im Ultraschallbad behandelt. Der Niederschlag wurde filtriert, in 10 ml 0.1N NaOH gelöst und filtriert. Das Filtrat stellte man mit 0.1N HCl auf pH = 6. Der Niederschlag wurde abgenutscht, mit Wasser gewaschen und getrocknet. Man erhielt 0.090 g (44%) 4-Amino-N-(2-azetidin-1-yl-6-ethylamino-pyrimidin-4-yl)-benzolsulfonamid als beige Kristalle; Smp.: 260-262°C.

**Beispiel 23**

4-Amino-N-(6-methylamino-2-pyrrolidin-1-yl-pyrimidin-4-yl)-benzolsulfonamid

0.58 g (0.00266 Mol) 4,6-Dichlor-2-pyrrolidin-1-yl-pyrimidin und 1.36 g (0.00539 Mol) N-(4-Sulfamoyl-phenyl)-acetamid Kaliumsalz wurden 8 Std. in 10 ml 1-Methyl-2-pyrrolidon bei 140°C gerührt. Dann destillierte man das Lösungsmittel am Hochvakuum ab, verteilte den Rückstand auf Essigester/Wasser und extrahierte. Die Wasserphase wurde mit 4N HCl sauer gestellt und mit Essigester extrahiert. Beide organische Phase wurden vereinigt und eingeengt. Der Rückstand wurde aus wenig Methanol umkristallisiert und die Mutterlauge wurde über Kieselgel mit Cyclohexan/Essigester 2:1 als Laufmittel chromatographiert. Man erhielt total 0.62 g (66%) N-[4-(6-Chlor-2-pyrrolidin-1-yl-pyrimidin-4-ylsulfamoyl)-phenyl]-acetamid als weissliche Kristalle; Smp. 229-233°C.

0.62 g (0.00175 Mol) N-[4-(6-Chlor-2-pyrrolidin-1-yl-pyrimidin-4-ylsulfamoyl)-phenyl]-acetamid wurden in 20 ml Ethanol gelöst, mit 1.36 g (0.0201 Mol) Methylamin Hydrochlorid und 5.3 ml (0.038 Mol) Triethylamin versetzt und in einem Autoklaven 17 Std. bei 140°C gerührt. Man befreite vollständig von den Lösungsmitteln, verteilte auf Essigester/Wasser und nutschte ab. Man erhielt 0.30 g (44%) N-[4-(6-Methylamino-2-pyrrolidin-1-yl-pyrimidin-4-ylsulfamoyl)-phenyl]-acetamid als beige Kristalle; Smp. 271-274°C.

0.30 g (0.00077 Mol) N-[4-(6-Methylamino-2-pyrrolidin-1-yl-pyrimidin-4-ylsulfamoyl)-phenyl]-acetamid wurden 3 Std. in 30 ml 1N wässrigem Natriumhydroxid am Rückfluss gekocht. Man kühlte ab und extrahierte mit Essigester. Aus der Wasserphase fielen dann Kristalle aus, die nach Abnutschen über Kieselgel mit Essigester/Ethanol 9:1 als Laufmittel chromatographiert wurden. Man erhielt 0.082 g (30%) 4-Amino-N-(6-methylamino-2-pyrrolidin-1-yl-pyrimidin-4-yl)-benzolsulfonamid als beige Kristalle; Smp. 299-301°C.

**Beispiel 24**

4-Amino-N-(6-amino-2-mercapto-pyrimidin-4-yl)-benzolsulfonamid

1 g (0.0043 Mol) 4-Acetamino-benzolsulfochlorid wurde in 20 ml Pyridin gelöst und mit 0.64 g (0.0045 Mol) 4,6-Diamino-pyrimidin-2-thiol versetzt. Man rührte 2 Std. bei Raumtemperatur, goss auf 30 ml Wasser, nutschte den Niederschlag ab und löste in 40 ml 1N NaOH. Man kochte 2 Std. am Rückfluss, filtrierte das Reaktionsgemisch, versetzte das Filtrat mit 1N HCl bis pH=6 und filtriert erneut vom ausgefallenen Niederschlag ab. Dieser wurde mit reichlich Wasser gewaschen und getrocknet. Man erhielt 0.96 g (75%) 4-Amino-N-(6-amino-2-mercapto-pyrimidin-4-yl)-benzolsulfonamid als gelbliche Kristalle; Smp.: 232-234°C.

**Beispiel 25**

4-Amino-N-(2-amino-6-methoxv-pyrimidin-4-yl)-benzolsulfonamid

0.27 g (0.0009 Mol) 4-Amino-N-(2-amino-6-chlor-pyrimidin-4-yl)-benzolsulfonamid wurden in einer Lösung von 0.23 g Natrium in 20 ml Methanol gelöst und 9 Std. bei 150°C in einem Autoklaven gerührt. Man destillierte das Lösungsmittel ab, gab den Rückstand auf Wasser und stellte mit 1N HCl den pH-Wert auf 4-5. Der dabei ausgefallene Niederschlag wurde abgenutscht, mit Wasser gewaschen und getrocknet. Die erhaltenen Kristalle verrührte man in 20 ml Methanol, filtrierte erneut ab und trocknete am Hochvakuum. Man erhielt 0.25 g (94%) 4-Amino-N-(2-amino-6-methoxy-pyrimidin-4-yl)-benzolsulfonamid als beige Kristalle; Smp.: > 250°C (Zers.).

**Beispiel 26**

4-Amino-N-(4,6-bis-methylamino-pyrimidin-2-yl)-benzolsulfonamid

0.25 g (0.0008 Mol) 4-Amino-N-(4,6-dimethoxy-pyrimidin-2-yl)-benzolsulfonamid wurde in 20 ml 8M Methylamin in Ethanol gelöst und 21 Std. in einem Autoklaven bei 130°C gerührt. Danach wurde das Reaktionsgemisch eingeengt, der Rückstand in 5 ml Ethanol suspendiert, im Ultraschallbad behandelt und der Niederschlag abfiltriert. Diesen löste man in 5 ml 1N NaOH, filtrierte die Lösung und stellte den pH-Wert mit 1N HCl auf 5. Vom dabei ausgefallenen Niederschlag wurde abfiltriert und dieser getrocknet. Man erhielt 0.1 g (42%) 4-Amino-N-(4,6-bis-methylaminopyrimidin-2-yl)-benzolsulfonamid als gelbe Kristalle; Smp.: 262-263°C.

**Beispiel 27**

N-(2,6-Bis-methylamino-pyrimidin-4-yl)-4-chlor-benzolsulfonamid

0.39 g (0.0022 Mol) (4,6-Dichlor-pyrimidin-2-yl)-methylamin und 1.0 g (0.0044 Mol) 4-Chlor-benzolsulfonamid Kaliumsalz wurden 8 Std. in 10 ml 1-Methyl-2-pyrrolidon bei 150°C gerührt. Dann destillierte man das Lösungsmittel am Hochvakuum ab, verteilte den Rückstand auf Essigester/Wasser und extrahierte. Die Wasserphase wurde mit Natriumchlorid gesättigt, kurz am Vakuum abgedampft, mit 4N HCl sauer gestellt und mit Dichlormethan extrahiert. Man brachte beide Extrakte zusammen und chromatographierte über Kieselgel mit Cyclohexan/Essigester 1:1 als Laufmittel. Man erhielt 0.62 g (85%) 4-Chlor-N-(6-chlor-2-methylamino-pyrimidin-4-yl)-benzolsulfonamid als weisse Kristalle; Smp. 196-198°C.

0.62 g (0.0019 Mol) 4-Chlor-N-(6-chlor-2-methylamino-pyrimidin-4-yl)-benzolsulfonamid wurden in 10 ml Ethanol gelöst, mit 1.67 g (0.025 Mol) Methylamin Hydrochlorid und 6.2 ml (0.044 Mol) Triethylamin versetzt und in einem Autoklaven 18 Std. bei 140°C gerührt. Man befreite vollständig von den Lösungsmitteln, verteilte den Rückstand auf Essigester/Wasser und nutschte die unlöslichen Anteile ab. Der Filterkuchen wurde in Methanol/Diethylether umkristallisiert. Man erhielt 0.28 g (45%) N-(2,6-Bis-methylamino-pyrimidin-4-yl)-4-chlor-benzolsulfonamid als weisse Kristalle; Smp. 272-273°C.

**Beispiel 28**

N-(2,6-Bis-methylamino-pyrimidin-4-yl)-4-tert-butyl-benzolsulfonamid

0.39 g (0.0022 Mol) (4,6-Dichlor-pyrimidin-2-yl)methyl-amin und 1.1 g (0.0044 Mol) 4-tert-Butyl-benzolsulfonamid Kaliumsalz wurden 8 Std. in 10 ml 1-Methyl-2-pyrrolidon bei 150°C gerührt. Dann destillierte man das Lösungsmittel am Hochvakuum ab, verteilte den Rückstand auf Essigester/Wasser und extrahierte. Die Wasserphase wurde mit Natriumchlorid gesättigt, am Vakuum entgast und mit 4N HCl sauer gestellt, wobei ein Niederschlag ausfiel. Man nutschte ab, brachte das Extrakt und den Feststoff zusammen und chromatographierte über Kieselgel mit Cyclohexan/Essigester 2:1 als Laufmittel. Das Produkt wurde aus Essigester/n-Hexan umkristallisiert. Man erhielt 0.57 g (73%) 4-tert-Butyl-N-(6-chlor-2-methylamino-pyrimidin-4-yl)-benzolsulfonamid als weisse Kristalle; Smp. 118-137°C (Zers.).

0.47 g (0.0013 Mol) 4-tert-Butyl-N-(6-chlor-2-methylamino-pyrimidin-4-yl)-benzolsulfonamid wurden in 10 ml Ethanol gelöst, mit 1.3 g (0.019 Mol) Methylamin Hydrochlorid und 4.7 ml (0.034 Mol) Triethylamin versetzt und in einem Autoklaven 18 Std. bei 140°C gerührt. Man befreite vollständig von den Lösungsmitteln, verteilte den Rückstand auf Essigester/Wasser und nutschte die unlöslichen Anteile ab. Der Filterkuchen wurde in Methanol/Diethylether umkristallisiert. Man erhielt 0.15 g (33%) N-(2,6-Bis-methylamino-pyrimidin-4-yl)-4-tert-butyl-benzolsulfonamid als weisse

Kristalle; Smp. 316-318°C.

**Beispiel 29**

N-(2,6-Bis-methylamino-pyrimidin-4-yl)-4-fluor-benzolsulfonamid

0.46 g (0.0026 Mol) (4,6-Dichlor-pyrimidin-2-yl)-methylamin und 1.1 g (0.0051 Mol) 4-Fluor-benzolsulfonamid Kaliumsalz wurden 8 Std. in 10 ml 1-Methyl-2-pyrrolidon bei 150°C gerührt. Dann destillierte man das Lösungsmittel am Hochvakuum ab, verteilte den Rückstand auf Essigester/Wasser und extrahierte. Die Wasserphase wurde mit Natriumchlorid gesättigt, kurz am Vakuum abgedampft und mit 4N HCl sauer gestellt, wobei ein Niederschlag ausfiel. Man nutschte ab, brachte das Extrakt und den Feststoff zusammen und chromatographierte über Kieselgel mit Cyclohexan/Essigester 2:1 als Laufmittel. Das Produkt wurde aus Essigester/n-Hexan umkristallisiert. Man erhielt 0.46 g (56%) N-(6-Chlor-2-methylamino-pyrimidin-4-yl)-4-fluor-benzolsulfonamid als weisse Kristalle; Smp. 121-123°C.

0.36 g (0.0011 Mol) N-(6-Chlor-2-methylamino-pyrimidin-4-yl)-4-fluorbenzolsulfonamid wurden in 10 ml Ethanol gelöst, mit 1.2 g (0.018 Mol) Methylamin Hydrochlorid und 4.0 ml (0.029 Mol) Triethylamin versetzt und in einem Autoklaven 17 Std. bei 140°C gerührt. Man befreite vollständig von den Lösungsmitteln, verteilte den Rückstand auf Essigester/Wasser, nutschte die unlöslichen Anteile ab und extrahierte. Man brachte das Extrakt und den Feststoff zusammen und kristallisierte aus Methanol/ Diethylether um. Man erhielt 0.15 g (43%) N-(2,6-Bis-methylaminopyrimidin-4-yl)-4-fluor-benzolsulfonamid als weisse Kristalle; Smp. 261-263°C.

**Beispiel 30**

N-(2,6-bis-methylamino-pyrimidin-4-yl)-benzolsulfonamid

5.38 g (0.0275 mol) Benzolsulfonamid Kaliumsalz und 2.45 g (0.0138 Mol) 2-Methylamino-4,6-dichlor-pyrimidin wurden in 22 ml 1-Methyl-2-pyrrolidon suspendiert und 24 Std. bei 150°C gerührt. Das Lösungsmittel wurde am Hochvakuum abgezogen und der Rückstand in 250 ml Wasser suspendiert. Man extrahierte dreimal mit je 100 ml Essigester und wusch die vereinigten organischen Phasen mit 200 ml ges. $NaHCO_3$. Die vereinigten wässrigen Phasen wurden mit 3N HCl sauergestellt und der dabei ausfallende Niederschlag abgenutscht. Man chromatographierte auf Kieselgel mit Dichlormethan/Methanol 99:1-> 98:2. Die nach der Chromatographie erhaltenen gelblichen Kristalle wurden in 70 ml 1N NaOH suspendiert, die Suspension filtriert und das klare Filtrat mit 1N HCl auf pH 3.5 gestellt. Der dabei ausgefallene Niederschlag wurde isoliert und getrocknet. Man erhielt 1.9 g (46%) N-(2-methylamino-6-chlor-pyrimidin-4-yl)-benzolsulfonamid als farblose Kristalle; Smp.: 186-187°C.

0.25 g (0.00084 Mol) N-(2-methylamino-6-chlor-pyrimidin-4-yl)-benzolsulfonamid wurden in 20 ml 2M Methylamin in THF gelöst und 3 Std. bei 130°C in einem Autoklaven gerührt. Man befreite vom Lösungsmittel, löste den Rückstand in 25 ml 2N NaOH, filtrierte und stellte den pH-Wert des Filtrates mit 1N HCl auf 6. Der Niederschlag wurde abgenutscht, getrocknet und auf Kieselgel mit Dichlormethan/Methanol 95:5 chromatographiert. Man erhielt 0.09 g (36%) N-(2,6-bis-methylamino-pyrimidin-4-yl)-benzolsulfonamid als gelbliche Kristalle; Smp.: > 260°C (Zers.).

**Beispiel 31**

N-(2,6-Bis-methylamino-pyrimidin-4-yl)-4-methyl-benzolsulfonamid

0.30 g (0.00097 Mol) N-(2,6-Dimethoxy-pyrimidin-4-yl)-4-methylbenzolsulfonamid wurden in 30 ml 33 prozentiger ethanolischer Methylamin gelöst und für 24 Std. bei 140°C in einem Autoklav gerührt. Man kühlte ab und nutschte ab. Man erhielt 0.125 g (42%) N-(2,6-Bismethylamino-pyrimidin-4-yl)-4-methyl-benzolsulfonamid als graue Kristalle; Smp. 270-272°C.

**Beispiel 32**

N-(2,6-Bis-methylamino-pyrimidin-4-yl)-3-chlor-benzolsulfonamid

0.24 g (0.00135 Mol) (4,6-Dichlor-pyrimidin-2-yl)-methylamin und 0.62 g (0.0027 Mol) 3-Chlor-benzolsulfonamid Kaliumsalz wurden 8 Std. in 10 ml 1-Methyl-2-pyrrolidon bei 150°C gerührt. Dann destillierte man das Lösungsmittel am Hochvakuum ab, verteilte den Rückstand auf Essigester/Wasser und extrahierte. Die Wasserphase wurde mit 4N HCl sauer gestellt und mit Dichlormethan extrahiert. Der Rückstand wurde über Kieselgel mit Cyclohexan/Essigester 2:1 als Laufmittel chromatographiert. Man erhielt 0.24 g (53%) 3-Chlor-N-(6-chlor-2-methylamino-pyrimidin-4-yl)-benzolsulfonamid als gelbe Kristalle; Smp. 120-130°C (Zers.).

0.10 g (0.00030 Mol) 3-Chlor-N-(6-chlor-2-methylamino-pyrimidin-4-yl)-benzolsulfonamid wurden in 5 ml EtOH gelöst, mit 0.27 g (0.004 Mol) Methylamin Hydrochlorid und 1 ml (0.007 Mol) Triethylamin versetzt und in einem Autoklaven 17 Std. bei 145°C gerührt. Man verteilte das ganze Reaktionsgemisch auf Essigester/Wasser und extrahierte. Der Rückstand wurde aus MeOH umkristallisiert. Man erhielt 0.04 g (41%) N-(2,6-Bis-methylamino-pyrimidin-4-yl)-3-chlor-benzolsulfonamid als weisse Kristalle; Smp. 167-168°C.

**Beispiel 33**

N-(2,6-Bis-methylamino-pyrimidin-4-yl)-3-trifluormethylbenzolsulfonamid

0.27 g (0.00155 Mol) (4,6-Dichlor-pyrimidin-2-yl)methyl-amin und 0.82 g (0.0031 Mol) 3-Trifluormethyl-benzolsulfonamid Kaliumsalz wurden 8 Std. in 10 ml 1-Methyl-2-pyrrolidon bei 150°C gerührt. Dann destillierte man das Lösungsmittel am Hochvakuum ab, verteilte den Rückstand auf Essigester/Wasser und extrahierte. Die Wasserphase wurde mit 4N HCl sauer gestellt und mit Dichlormethan extrahiert. Der Rückstand wurde über Kieselgel mit Cyclohexan/Essigester 2:1 als Laufmittel chromatographiert. Man erhielt 0.26 g (69%) N-(6-Chlor-2-methylaminopyrimidin-4-yl)-3-trifluormethyl-benzolsulfonamid als weiss-beige Kristalle; Smp. 190-193°C.

0.10 g (0.00027 Mol) N-(6-Chlor-2-methylamino-pyrimidin-4-yl)-3-trifluormethyl-benzolsulfonamid wurden in 5 ml EtOH gelöst, mit 0.27 g (0.004 Mol) Methylamin Hydrochlorid und 1 ml (0.007 Mol) Triethylamin versetzt und in einem Autoklaven 17 Std. bei 145°C gerührt. Man verteilte das ganze Reaktionsgemisch auf Essigester/Wasser und extrahierte. Der Rückstand wurde in MeOH umkristallisiert. Man erhielt 0.055 g (56%) N-(2,6-Bis-methylamino-pyrimidin-4-yl)-3-trifluormethyl-benzolsulfonamid als weisse Kristalle; Smp. 234-235°C.

**Beispiel 34**

4-Amino-N-(3,5-diamino-phenyl)-benzolsulfonamid

0.105 g (0.00033 Mol) N-[4-(3,5-Diamino-phenylsulfamoyl)-phenyl]-acetamid wurden in 6.5 ml 1N NaOH gelöst und 1.5 Std. am Rückfluss gekocht. Das Reaktionsgemisch wurde mit 50 ml ges. Ammoniumchloridlösung versetzt und mit je 100 ml Essigester zweimal extrahiert. Die vereinigten organischen Phasen wurden mit ges. Kochsalzlösung gewaschen und über $MgSO_4$ getrocknet. Nach Entfernung des Lösungsmittels chromatographierte man den Rückstand auf Aluminiumoxid (neutral, Aktivität I) mit zuerst 5% dann 10% Methanol in Dichlormethan. Man erhielt 0.05 g (55%) 4-Amino-N-(3,5-diamino-phenyl)-benzolsulfonamid als beigen Feststoff; Smp.: 188-190°C.

**Beispiel 35**

4-Amino-N-(3,5-bis-methylamino-phenyl)-benzolsulfonamid Hydrochlorid

0.24 g (0.001 Mol) N-(3-Acetylamino-5-nitro-phenyl)-acetamid wurden in 20 ml THF suspendiert, mit 0.092 g (0.0023 Mol) NaH und 10 ml DMF versetzt und 1.5 Std. bei Raumtemperatur gerührt. Dann gab man 0.29 ml (0.0046 mol) Methyliodid zu und rührte weitere 48 Std. Anschliessend wurde das Lösungsmittel abdestilliert, der Rückstand in 100 ml Wasser aufgenommen, viermal mit je 80 ml Essigester extrahiert, die vereinigten organischen Phasen mit ges. Kochsalzlösung gewaschen und über Natriumsulfat getrocknet. Nach Entfernen des Lösungsmittels chromatographierte man auf Kieselgel mit 3% Methanol in Dichlormethan. Man erhielt 0.2 g (75%) N-[3-(Acetyl-methyl-amino)-5-nitro-phenyl]-N-methyl-acetamid als braunes Oel. MS (EI): me/e = 265 ($C_{12}H_{15}N_3O_4^+$).

0.19 g (0.00072 Mol) N-[3-(Acetyl-methyl-amino)-5-nitro-phenyl]-N-methyl-acetamid wurden in 15 ml Ethanol gelöst, mit 0.019 g Pd/C (10%) versetzt und 2 Std. bei Raumtemperatur mit Wasserstoffgas hydriert. Der Katalysator wurde abfiltriert, das Lösungsmittel abdestilliert und der Rückstand auf Kieselgel mit Essigester chromatographiert. Man erhielt 0.16 g (94%) N-[3-(Acetyl-methyl-amino)-5-amino-phenyl]-N-methyl-acetamid als weisse Kristalle; Smp.: 179-181°C.

0.15 g (0.00063 Mol) N-[3-(Acetyl-methyl-amino)-5-amino-phenyl]-N-methyl-acetamid wurden in 3 ml Pyridin gelöst, mit 0.154 g (0.00064 Mol) 4-Acetamino-benzolsulfochlorid versetzt und 16 Std. bei Raumtemperatur gerührt. Anschliessend wurde vom Lösungsmittel befreit, der Rückstand in 25 ml Wasser aufgenommen, viermal mit je 200 ml Essigester extrahiert und die vereinigten organischen Phasen mit ges. Kochsalzlösung gewaschen und über Natriumsulfat getrocknet. Nach Abzug des Lösungsmittels chromatographierte man den Rückstand auf Kieselgel mit zuerst 2% dann 5% Methanol in Dichlormethan. Man erhielt 0.15 g (55%) N-[3-(4-Acetylamino-phenylsulfonylamino)-5-(acetyl-methyl-amino)-phenyl]-N-methyl-acetamid als orangefarbenen, amorphen Feststoff. MS (ISN): me/e = 431 ($C_{20}H_{23}N_4O_5S^-$).

0.113 g (0.00026 Mol) N-[3-(4-Acetylamino-phenylsulfonylamino)-5-(acetyl-methyl-amino)-phenyl]-N-methyl-acetamid wurden in 10 ml 1N NaOH gelöst und 2 Std. am Rückfluss gekocht. Man neutralisierte mit 1N HCl, extrahierte mit Essigester und trocknete die organische Phase über Natriumsulfat. Nach Abzug des Lösungsmittels wurde der Rückstand in 4 ml Methanol gelöst und mit 3 ml 2M HCl versetzt. Nach Zugabe von 7-8 ml Essigester fiel das Produkt langsam aus. Es wurde abfiltriert und am Vakuum getrocknet. Man erhielt 0.085 g (74%) 4-Amino-N-(3,5-bis-methyl-amino-phenyl)-benzolsulfonamid Hydrochlorid (1:2.6) als rosafarbenen, amorphen Feststoff. MS (ISN): me/e = 305 ($C_{14}H_{17}N_4O_2S^-$).

**Beispiel 36**

4-Amino-N-(3-methylamino-phenyl)-benzolsulfonamid

1.3 g (0.0079 Mol) N-(3-Amino-phenyl)-N-methyl-acetamid wurden in 50 ml Pyridin gelöst, mit 1.94 g (0.0083 Mol) 4-Acetamino-benzolsulfochlorid versetzt und 18 Std. bei Raumtemperatur gerührt. Das Pyridin wurde abdestilliert, der Rückstand in Wasser suspendiert und abgenutscht. Man wusch mit reichlich Wasser und trocknete das Nutschengut am Hochvakuum. Man erhielt 2.7 g (94%) N-[4-[3-(Acetyl-methyl-amino)-phenylsulfamoyl]-phenyl]-acetamid als leicht gelbfarbenen Feststoff; Smp.: 258-260°C.

2.5 g (0.0069 Mol) N-[4-[3-(Acetyl-methyl-amino)-phenylsulfamoyl]-phenyl]-acetamid wurden in 150 ml 1N NaOH gelöst und 4 Std. am Rückfluss gekocht. Anschliessend stellte man den pH-Wert mit 1N HCl auf 4 und filtrierte vom ausgefallenen Niederschlag ab. Nach Trocknen wurde das Nutschengut auf Kieselgel mit 3% Methanol in Dichlormethan chromatographiert. Man erhielt 1.64 g (85%) 4-Amino-N-(3-methylamino-phenyl)-benzolsulfonamid als gelblichen Feststoff; Smp.: 134-135°C.

**Beispiel 37**

N-(3,5-Dimethoxy-phenyl)-3-trifluormethyl-benzolsulfonamid

0.31 g (0.002 Mol) 3,5-Dimethoxyanilin wurden in 10 ml Pyridin gelöst, mit 0.54 g (0.0022 Mol) 3-Trifluormethyl-benzolsulfochlorid versetzt und 2 Std. bei Raumtemperatur gerührt. Nach Abzug des Lösungsmittels wurde der Rückstand in Wasser aufgenommen, mit Essigester extrahiert, die organische Phase mit ges. Kochsalzlösung gewaschen und über Natriumsulfat getrocknet. Das Lösungsmittel wurde eingeengt und der Rückstand auf Kieselgel mit Hexan/Essigester 2:1 chromatographiert. Man erhielt 0.68 g (94%) N-(3,5-Dimethoxy-phenyl)-3-trifluormethylbenzolsulfonamid als weisse Kristalle; Smp.: 78-81°C.

**Beispiel 38**

4-Amino-N-(2,6-dibrom-pyridin-4-yl)-benzolsulfonamid

5.1 g (0.02 Mol) 4-Amino-2,6-dibrom-pyridin wurden in 100 ml Pyridin gelöst, mit 7.1 g (0.03 Mol) 4-Acetamino-benzolsulfochlorid versetzt und 16 Std. bei 60°C gerührt. Nach dem Abzug des Lösungsmittels wurde der Rückstand in 100 ml 1N HCl aufgenommen, zweimal mit je 100 ml Essigester extrahiert, die vereinigten organischen Phasen mit ges. Kochsalzlösung gewaschen und über Natriumsulfat getrocknet. Dann wurde vom Lösungsmittel befreit und am Hochvakuum getrocknet. Man erhielt 7.2 g (79%) N-[4-(2,6-Dibrom-pyridin-4-ylsulfamoyl)-phenyl]-acetamid als gelbe Kristalle; Smp.: >260°C (Zers.).

6.2 g (0.0138 Mol) N-[4-(2,6-Dibrom-pyridin-4-ylsulfamoyl)-phenyl]-acetamid wurden in 138 ml 1N NaOH gelöst und 2 Std. am Rückfluss gekocht. Nach dem Abkühlen stellte man mit 2N HCl auf pH 6 und filtrierte vom ausgefallenen Niederschlag ab. Das Nutschengut wurde mit reichlich Wasser gewaschen und getrocknet. Anschliessend chromatographierte man auf Kieselgel mit Essigester/Hexan 1:2 -> 1:1. Man erhielt 4.86 g (86%) 4-Amino-N-(2,6-dibrom-pyridin-4-yl)-benzolsulfonamid als beige Kristalle; Smp.: 220-222°C.

**Beispiel 39**

4-Amino-N-(2-chlor-6-methylamino-pyridin-4-yl)-benzolsulfonamid Dihydrochlorid

0.82 g (0.005 Mol) 4-Amino-2,6-dichlor-pyridin wurden in 25 ml Pyridin gelöst, mit 1.3 g (0.0055 Mol) 4-Acetamino-benzolsulfochlorid versetzt und 16 Std. bei 60°C gerührt. Nach dem Abzug des Lösungsmittels wurde der Rückstand in 50 ml 1N HCl aufgenommen, zweimal mit je 50 ml Essigester extrahiert, die vereinigten organischen Phasen mit ges. Kochsalzlösung gewaschen und über Natriumsulfat getrocknet. Dann wurde vom Lösungsmittel befreit und der Rückstand auf Kieselgel mit Essigester/Hexan 4:1 chromatographiert. Man erhielt 1.45 g (80%) N-[4-(2,6-Dichlor-pyridin-4-ylsulfamoyl)-phenyl]-acetamid als gelbe Kristalle; Smp.: > 246°C (Zers.).

1.25 g (0.0035 Mol) N-[4-(2,6-Dichlor-pyridin-4-ylsulfamoyl)-phenyl]-acetamid wurden in 35 ml 1N NaOH gelöst und 2 Std. am Rückfluss gekocht. Nach dem Abkühlen stellte man mit 2N HCl auf pH 6 und filtrierte vom ausgefallenen Niederschlag ab. Das Nutschengut wurde mit reichlich Wasser gewaschen und getrocknet. Anschliessend chromatographierte man auf Kieselgel mit Essigester/Hexan 1:2 -> 1:1. Man erhielt 1.04 g (93%) 4-Amino-N-(2,6-dichlor-pyridin-4-yl)-benzolsulfonamid als weisse Kristalle; Smp.: 209-211°C (Zers.).

0.11 g (0.00035 Mol) wurden in 25 ml flüssigem Methylamin in einem Autoklaven bei 130°C 72 Std. gerührt. Nach Abzug des Methylamins nahm man den Rückstand in 5 ml Dichlormethan/Methanol 1:1 auf, filtrierte und chromatographierte das Filtrat nach Abzug des Lösungsmittels auf Kieselgel mit Hexan/Essigester 3:1, 2:1 und schliesslich 1:1. Das als weisse, amorphe Festsubstanz erhaltene Produkt wurde in 2 ml Methanol gelöst, mit 2 ml 2N HCl/Methanol versetzt, mit 20 ml Diethylether verdünnt und abgenutscht. Man wusch mit reichlich Diethylether nach und trocknete das Nutschengut am Hochvakuum. Man erhielt 0.055 g (41%) 4-Amino-N-(2-chlor-6-methylamino-pyridin-4-yl)-benzolsulfonamid Dihydrochlorid (1:2) als weisse Kristalle; Smp.: > 215°C (Zers).

**Beispiel 40**

4-Amino-N-(2-brom-6-methylamino-pyridin-4-yl)-benzolsulfonamid Hydrochlorid

0.81 g (0.002 Mol) 4-Amino-N-(2,6-dibrom-pyridin-4-yl)-benzolsulfonamid wurden in 35 ml flüssigem Methylamin in einem Autoklaven 44 Std. bei 130°C gerührt. Man liess das Methylamin verdampfen, löste den Rückstand in Ethanol, versetzte mit 2 g Kieselgel, engte ein und chromatographierte den Rückstand auf Kieselgel mit zuerst Essigester/Hexan 1:2, dann Essigester pur. Man erhielt 0.63 g (88%) 4-Amino-N-(2-brom-6-methylamino-pyridin-4-yl)-benzolsulfonamid als beigen Schaum. 0.33 g (0.00092 Mol) davon wurden in 10 ml Methanol gelöst, mit 5 ml 2M HCl in Methanol versetzt, eingeengt und erneut mit 4 ml Methanol versetzt. Den erhaltenen Niederschlag nutschte man ab, wusch mit wenig Methanol nach und trocknete am Hochvakuum. Man erhielt 0.27 g (69%) 4-Amino-N-(2-brom-6-methylamino-pyridin-4-yl)-benzolsulfonamid Hydrochlorid (1:1.9) als weisse Kristalle; Smp.: 226-228°C (Zers.).

**Beispiel 41**

4-Amino-N-(2-brom-6-ethylamino-pyridin-4-yl)-benzolsulfonamid

5.1 g (0.0125 Mol) 4-Amino-N-(2,6-dibrom-pyridin-4-yl)-benzolsulfonamid wurden in 100 ml flüssigem Ethylamin in einem Autoklaven 24 Std. bei 150°C gerührt. Man liess das Ethylamin verdampfen, löste den Rückstand in Ethanol, versetzte mit 5 g Kieselgel, engte ein und chromatographierte den Rückstand auf Kieselgel mit zuerst Essigester/Hexan 1:2 und dann 1:1. Man erhielt 2.15 g eines braunen Oels, das in 200 ml 25%iger HCl suspendiert wurde. Man nutschte ab, stellte das Filtrat mit 2N NaOH auf pH=8 und isolierte den ausgefallenen Niederschlag. Nach Trocknen am Hochvakuum erhielt man 0.96 g (21%) 4-Amino-N-(2-brom-6-ethylamino-pyridin-4-yl)-benzolsulfonamid als beige Kristalle; Smp.: > 85°C (Zers.).

**Beispiel 42**

4-Amino-N-(2,6-bis-methylamino-pyridin-4-yl)-benzolsulfonamide Hydrochlorid

0.90 g (0.002 Mol) 4-Amino-N-(2,6-dibrom-pyridin-4-yl)-benzolsulfonamid wurden in 35 ml flüssigem Methylamin in einem Autoklaven 16 Std. bei 160°C gerührt. Man liess das Methylamin verdampfen, löste den Rückstand in Ethanol, versetzte mit 1 g Kieselgel, engte ein und chromatographierte den Rückstand auf Kieselgel mit zuerst Essigester/Hexan 1:1 und dann Essigester pur. Man erhielt 0.34 g eines braunen Oels, das in 100 ml 1N NaOH suspendiert wurde. Man nutschte ab, stellte das Filtrat mit 1N HCl auf pH=8 und isolierte den ausgefallenen Niederschlag. Nach Trocknen am Hochvakuum erhielt man 0.22 g (35%) 4-Amino-N-(2,6-bis-methylamino-pyridin-4-yl)-benzolsulfonamid als beige Kristalle; Smp.: > 280°C (Zers.). Diese wurden in 5 ml Methanol gelöst und mit 3 ml 2N methanolischer HCl versetzt. Vom ausgefallenen Niederschlag wurde abfiltriert und dieser getrocknet. Man erhielt 0.13 g 4-Amino-N-(2,6-bis-methylamino-pyridin-4-yl)-benzolsulfonamid Hydrochlorid (1:3) als leicht beige Kristalle; Smp.: 197°C (Zers.).

**Beispiel 43**

4-Amino-N-(2-ethylamino-6-methylamino-pyridin-4-yl)-benzolsulfonamid

2.5 g (0.007 Mol) 4-Amino-N-(2-brom-6-methylamino-pyridin-4-yl)-benzolsulfonamid wurden mit 80 ml Ethylamin 40 Std. in einem Autoklaven bei 160°C gerührt. Man liess das restliche Ethylamin verdampfen und chromatographierte den Rückstand auf Kieselgel mit Essigester/Hexan 1:2, dann 2:3 und schliesslich 1:1. Die dabei erhaltenen 0.67 g eines braunen Oels wurden in 200 ml 1N NaOH suspendiert, abgenutscht und das Filtrat mit 1N HCl neutralisiert. Man nutschte erneut ab, sättigte das Filtrat mit NaCl und extrahierte mit Essigester. Die organische Phase wurde getrocknet. Man erhielt 0.055 g (2.5%) 4-Amino-N-(2-ethylamino-6-methylamino-pyridin-4-yl)-benzolsulfonamid als hellbraune Kristalle: MS(ISN): me/e = 320 ($C_{14}H_{18}N_5O_2S^-$).

**Beispiel 44**

4-Amino-N-(2-dimethylamino-6-methylamino-pyridin-4-yl)-benzolsulfonamid

2.5 g (0.007 Mol) 4-Amino-N-(2-brom-6-methylamino-pyridin-4-yl)-benzolsulfonamid wurden mit 80 ml Dimethylamin 70 Std. in einem Autoklaven bei 160°C gerührt. Man liess das restliche Dimethylamin verdampfen und chromatographierte den Rückstand auf Kieselgel mit Essigester/Hexan 1:1, dann 2:1 und schliesslich 3:1. Die polaren Fraktionen wurden noch zweimal auf Kieselgel mit Essigester/Hexan 2:3 und 1:1 als Laufmittel chromatographiert. Man erhielt 0.175 g (8%) 4-Amino-N-(2-dimethylamino-6-methylamino-pyridin-4-yl)-benzolsulfonamid als beige Kristalle: MS(ISP): me/e = 322 ($C_{14}H_{20}N_5O_2S^+$).

**Beispiel 45**

4-Amino-N-(2,6-bis-ethylamino-pyridin-4-yl)-benzolsulfonamid

5.1 g (0.0125 Mol) 4-Amino-N-(2,6-dibrom-pyridin-4-yl)-benzolsulfonamid wurden in 100 ml flüssigem Ethylamin in einem Autoklaven 24 Std. bei 150°C gerührt. Man liess das Ethylamin verdampfen, löste den Rückstand in Ethanol, versetzte mit 5 g Kieselgel, engte ein und chromatographierte den Rückstand auf Kieselgel mit zuerst Essigester/Hexan 1:2, dann 1:1 und schliesslich 2:1. Man erhielt 1.16 g eines braunen Feststoffes, welche in 500 ml 1N NaOH suspendiert wurden. Man filtrierte ab, stellte das Filtrat auf pH=8, filtrierte erneut ab und extrahierte das Filtrat mit Essigester. Man vereinigte das Nutschengut der letzten Filtration mit der Essigesterphase, engte ein und chromatographierte den Rückstand auf Kieselgel mit Essigester/Hexan 2:1. Man erhielt 0.22 g braungefärbten Feststoff, der in 20 ml 1N NaOH suspendiert wurde. Es wurde erneut filtriert, das Filtrat mit 1N HCl auf pH=8 gestellt und das ausgefallenen Produkt abgenutscht. Man erhielt 0.14 g (3%) 4-Amino-N-(2,6-bis-ethylamino-pyridin-4-yl)-benzolsulfonamid als beige Kristalle; Smp.: 212-215°C (Zers.).

**Beispiel 46**

4-Amino-N-(2-methylamino-pyridin-4-yl)-benzolsulfonamid

0.5 g (0.0014 Mol) 4-Amino-N-(2-brom-6-methylamino-pyridin-4-yl)-benzolsulfonamid wurden in 25 ml Ethanol gelöst, mit 0.05 g Pd/C versetzt und 1 Std. unter Normaldruck mit Wasserstoffgas hydriert. Man filtrierte vom Katalysator, engte ein und löste den Rückstand in 10 ml 1N NaOH, filtrierte und stellte dann das Filtrat mittels 1N HCl auf pH=8. Der langsam ausfallende Niederschlag wurde abgenutscht, nachgewaschen und am Hochvakuum getrocknet. Man erhielt 0.22 g (57%) 4-Amino-N-(2-methylamino-pyridin-4-yl)-benzolsulfonamid als weisse Kristalle; Smp.: > 261°C (Zers.).

**Beispiel 47**

4-Amino-N-(2,6-bis-dimethylamino-pyridin-4-yl)-benzolsulfonamid

0.81 g (0.002 Mol) 4-Amino-N-(2,6-dibrom-pyridin-4-yl)-benzolsulfonamid wurden in 30 ml Dimethylamin in einem Autoklaven 45 Std. bei 160°C gerührt. Man liess das überschüssige Dimethylamin verdampfen, löste den Rückstand in einer Mischung aus Methanol und Essigester, versetzte mit 1 g Kieselgel, engte ein und chromatographierte den Rückstand auf Kieselgel mit zuerst Essigester/Hexan 1:3 und dann 1:2. Nach Trocknen am Hochvakuum erhielt man 0.67 g (100%) 4-Amino-N-(2,6-bis-dimethylamino-pyridin-4-yl)-benzolsulfonamid als beige Kristalle; Smp.: 157-160°C (Zers.).

**Beispiel 48**

N-(2,6-Bis-methylamino-pyridin-4-yl)-4-chlor-benzolsulfonamid Dihydrochlorid

3.0 g (0.012 Mol) 4-Amino-2,6-dibrom-pyridin wurden in 60 ml Pyridin gelöst, mit 2.76 g (0.013 Mol) 4-Chlorbenzolsulfochlorid versetzt und 5 Std. bei 60°C gerührt. Nach Abzug des Lösungsmittels wurde der Rückstand in 100 ml 1N HCl aufgenommen, zweimal mit je 100 ml Essigester extrahiert, die vereinigten organischen Phasen mit ges. Kochsalzlösung gewaschen und über Natriumsulfat getrocknet. Es wurde vom Lösungsmittel befreit und der Rückstand auf Kieselgel mit Essigester/Hexan 1:4 und dann 1:1 chromatographiert. Man erhielt 4.4 g (87%) 4-Chlor-N-(2,6-dibrom-pyridin-4-yl)-benzolsulfonamid als gelbe Kristalle. Zu analytischen Zwecken wurden 0.2 g (0.0005 Mol) aus tert.-Butylmethylether umkristallisiert; Smp.: 203-205°C.

0.50 g (0.0017 Mol) 4-Chlor-N-(2,6-dibrom-pyridin-4-yl)-benzolsulfonamid wurden in 35 ml flüssigem Methylamin mit 0.025 g Cu-Pulver in einem Autoklaven 18 Std. bei 80°C gerührt. Man liess das Methylamin verdampfen, löste den Rückstand in 50 ml Wasser, stellte den pH-Wert auf 8 und extrahierte dreimal mit je 100 ml Essigester. Die vereinigten organischen Phasen wurden mit ges. Kochsalzlösung gewaschen und über Natriumsulfat getrocknet. Man engte ein und suspendierte den Rückstand in 1N NaOH. Nach Filtration wurde das klare Filtrat mit NaCl gesättigt und mit 1N HCl angesäuert bis pH 1. Dabei fiel ein beiger Niederschlag aus, der isoliert wurde. Man erhielt 0.24 g (50%) N-(2,6-Bis-methylamino-pyridin-4-yl)-4-chlor-benzolsulfonamid Dihydrochlorid als beige Kristalle; Smp.: > 170°C (Zers.).

**Beispiel 49**

3-Chlor-N-(2,6-bis-methylamino-pyridin-4-yl)-benzolsulfonamid

2.0 g (0.0079 Mol) 4-Amino-2,6-dibrom-pyridin wurden in 24 ml Pyridin gelöst, mit 2.5 g (0.012 Mol) 3-Chlorbenzolsulfochlorid versetzt und 5 Std. bei 60°C gerührt. Nach dem Abzug des Lösungsmittels wurde der Rückstand in 100 ml 1N HCl aufgenommen, zweimal mit je 100 ml Essigester extrahiert, die vereinigten organischen Phasen mit ges. Kochsalzlösung gewaschen und über Natriumsulfat getrocknet. Es wurde vom Lösungsmittel befreit und der Rückstand auf Kieselgel mit Essigester/Hexan 1:2, dann 1:1 und schliesslich 2:1 chromatographiert. Man erhielt 2.12 g (63%) 3-Chlor-N-(2,6-dibrom-pyridin-4-yl)-benzolsulfonamid als gelbe Kristalle; Smp.: 187-189°C.

1.0 g (0.0023 Mol) 3-Chlor-N-(2,6-dibrom-pyridin-4-yl)-benzolsulfonamid wurden in 35 ml flüssigem Methylamin in einem Autoklaven 18 Std. bei 160°C gerührt. Man liess das Methylamin verdampfen, löste den Rückstand in Wasser, stellte den pH-Wert auf 8 und extrahierte dreimal mit je 100 ml Essigester. Die vereinigten organischen Phasen wurden mit ges. Kochsalzlösung gewaschen und über Natriumsulfat getrocknet. Das Lösungsmittel wurde eingeengte und der Rückstand auf Kieselgel mit zuerst Essigester/Hexan 2:1, dann Essigester pur und zuletzt Essigester/Methanol 1:1 chromatographiert. Anschliessend wurde die Produktfraktion erneut mit Hexan/Essigester 1:1 auf Kieselgel chromatographiert. Man erhielt 0.165 g (22%) 3-Chlor-N-(2,6-bis-methylamino-pyridin-4-yl)-benzolsulfonamid als leicht rötlichgefärbte Kristalle. 0.13 g (0.0004 Mol) davon wurden in 20 ml 1N NaOH suspendiert, filtriert und das klare Filtrat mit 1N HCl auf pH 8 gestellt. Der dabei ausgefallene Niederschlag wurde abgenutscht und am Hochvakuum getrocknet. Man erhielt 0.11 g (15%) 3-Chlor-N-(2,6-bis-methylamino-pyridin-4-yl)-benzolsulfonamid; Smp.: 133°C (Zers.).

**Beispiel 50**

N-(2,6-Bis-methylamino-pyridin-4-yl)-3-trifluormethylbenzolsulfonamid Dihydrochlorid

3.0 g (0.0012 Mol) 4-Amino-2,6-dibrom-pyridin wurden in 60 ml Pyridin gelöst, mit 2.1 ml (0.013 Mol) 3-Trifluormethyl-benzolsulfochlorid versetzt und 5 Std. bei 60°C gerührt. Nach dem Abzug des Lösungsmittels wurde der Rückstand in 100 ml Wasser aufgenommen, zweimal mit je 100 ml Diethylether extrahiert, die vereinigten organischen Phasen mit ges. Kochsalzlösung gewaschen und über Natriumsulfat getrocknet. Es wurde vom Lösungsmittel befreit und der Rückstand auf Kieselgel mit Essigester/Hexan 1:2 und dann 1:1 chromatographiert. Man erhielt 4.77 g (87%) N-(2,6-Dibrom-pyridin-4-yl)-3-trifluormethyl-benzolsulfonamid als hellgelbe Kristalle. Durch Umkristallisation in tert.-Butylmethylether wurde eine analytisch reine Probe erhalten; Smp.: 149-151°C.

0.50 g (0.0011 Mol) N-(2,6-Dibrom-pyridin-4-yl)-3-trifluormethylbenzolsulfonamid wurden in 35 ml flüssigem Methylamin mit 0.022 g Cu-Pulver in einem Autoklaven 18 Std. bei 80°C gerührt. Man liess das Methylamin verdampfen, löste den Rückstand in 50 ml Wasser, stellte den pH-Wert mit 1N HCl auf 8 und extrahierte dreimal mit je 100 ml Essigester. Die vereinigten organischen Phasen wurden mit ges. Kochsalzlösung gewaschen und über Natriumsulfat getrocknet. Man engte ein und chromatographierte den Rückstand auf Kieselgel mit Essigester/Hexan 1:9, 1:4, 1:1 und 1:0. Die mit reinem Essigester erhaltene Fraktion wurde in 1N NaOH suspendiert. Nach Filtration wurde das klare

Filtrat mit NaCl gesättigt und mit 1N HCl angesäuert bis pH 1. Dabei fiel ein beiger Niederschlag aus, der isoliert wurde. Man erhielt 0.094 g (15%) N-(2,6-Bis-methylamino-pyridin-4-yl)-3-trifluormethyl-benzolsulfonamid Dihydrochlorid als beige Kristalle; Smp.: >227°C (Zers.).

**Beispiel 51**

4-Amino-N-(2-methyl-6-methylamino-pyridin-4-yl)-benzolsulfonamid

1.54 g (0.0082 Mol) 4-Amino-2-brom-6-methyl-pyridin wurden in 25 ml Pyridin gelöst, mit 2.9 g (0.0124 Mol) 4-Acetamino-benzolsulfochlorid versetzt und 16 Std. bei 60°C gerührt. Nach dem Abzug des Lösungsmittels wurde der Rückstand auf Kieselgel mit Essigester als Laufmittel chromatographiert. Die Produkt enthaltenen Fraktionen wurden vom Lösungsmittel befreit und am Hochvakuum getrocknet. Man erhielt 2.17 g (69%) N-[4-(2-Brom-6-methyl-pyridin-4-ylsulfamoyl)-phenyl]-acetamid als farblose Kristalle; Smp.: 262-264°C (Zers.).

1.15 g (0.003 Mol) N-[4-(2-Brom-6-methyl-pyridin-4-ylsulfamoyl)-phenyl]-acetamid wurden in 15 ml 2N NaOH gelöst und 1 Std. am Rückfluss gekocht. Nach dem Abkühlen stellte man mit 2N HCl auf pH 5 und filtrierte den ausgefallenen, farblosen Niederschlag ab. Das Nutschengut wurde mit reichlich Wasser gewaschen und getrocknet. Man erhielt 0.95 g (93%) 4-Amino-N-(2-brom-6-methyl-pyridin-4-yl)-benzolsulfonamid als farblose Kristalle; Smp.: >110°C (Zers.).

0.94 g (0.00275 Mol) 4-Amino-N-(2-brom-6-methyl-pyridin-4-yl)-benzolsulfonamid wurden in 50 ml 8M Methylamin in Ethanol in einem Autoklaven 40 Std. bei 135°C gerührt. Man liess das Methylamin verdampfen, löste den Rückstand in Ethanol, versetzte mit 2 g Kieselgel, engte ein und chromatographierte den Rückstand auf Kieselgel mit zuerst Essigester/Hexan 1:1, dann 9:1. Man erhielt 0.39 g (48%) 4-Amino-N-(2-methyl-6-methylamino-pyridin-4-yl)-benzolsulfonamid als farblose Kristalle; Smp.: 173-175°C. 0.081 g (0.00028 Mol) davon wurden aus Methanol, Diethylether und Hexan umkristallisiert, nach dem Trocknen am Hochvakuum in 3 ml Methanol gelöst und mit 2.8 ml 0.1N NaOH versetzt. Das Methanol wurde abdestilliert und der Rückstand zweimal gefriergetrocknet. Man erhielt 0.084 g (97%) 4-Amino-N-(2-methyl-6-methylamino-pyridin-4-yl)-benzolsulfonamid Natriumsalz (1:1) als weisse Kristalle. MS (ISP): me/e= 293 ($C_{13}H_{17}N_4O_2S^+$). 0.31 g (0.0011 mol) 4-Amino-N-(2-methyl-6-methylamino-pyridin-4-yl)-benzolsulfonamid wurden in 2 ml Methanol gelöst, mit 1 ml 2.4N HCl in Diethylether versetzt und vom Lösungsmittel befreit. Der Rückstand wurde in 1 ml Methanol gelöst und langsam auf heftig gerührten Diethylether getropft. Dabei fiel ein farbloser Niederschlag aus, der isoliert und am Hochvakuum getrocknet wurde. Man erhielt 0.33 g (87%) 4-Amino-N-(2-methyl-6-methylamino-pyridin-4-yl)-benzolsulfonamid Hydrochlorid (1:1.86) als weisse Kristalle; Smp.: > 170°C (Zers.).

**Beispiel 52**

4-Amino-N-(1H-indol-4-yl)-benzolsulfonamid

0.095 g (0.00072 Mol) 4-Amino-1H-indol wurden in 3 ml Pyridin gelöst, mit 0.20 g (0.00086 Mol) 4-Acetamino-benzolsulfochlorid versetzt und 16 Std. bei 60°C gerührt. Nach dem Abzug des Lösungsmittels wurde der Rückstand in 10 ml 1N HCl aufgenommen, zweimal mit je 20 ml Essigester extrahiert, die vereinigten organischen Phasen mit ges. Kochsalzlösung gewaschen und über Natriumsulfat getrocknet. Dann wurde vom Lösungsmittel befreit und am Hochvakuum getrocknet. Man erhielt 0.155 g (65%) N-[4-(1H-Indol-4-ylsulfamoyl)-phenyl]-acetamid als hell-graue Kristalle; Smp.: >260°C.

0.15 g (0.00045 Mol) N-[4-(1H-Indol-4-ylsulfamoyl)-phenyl]-acetamid wurden in 4 ml 1N NaOH gelöst und 1 Std. am Rückfluss gekocht. Nach dem Abkühlen stellte man mit 0.1N HCl auf pH 6 und filtrierte vom ausgefallenen Niederschlag ab. Das Nutschengut wurde mit reichlich Wasser gewaschen und getrocknet. Anschliessend chromatographierte man auf Kieselgel mit Essigester/Hexan 1:1. Man erhielt 0.085 g (66%) 4-Amino-N-(1H-indol-4-yl)-benzolsulfonamid als weissen amorphen Feststoff; Smp.: 215°C.

**Beispiel 53**

4-Amino-N-(2-methylamino-6-trifluormethyl-pyridin-4-yl)-benzolsulfonamid

0.15 g (0.00076 Mol) 4-Amino-2-chlor-6-trifluorpyridin wurden in 4 ml Pyridin gelöst, mit 0.26 g (0.0015 Mol) 4-Acetamino-benzolsulfochlorid versetzt und 20 Std. bei 60°C gerührt. Nach dem Abzug des Lösungsmittels wurde der Rückstand in 10 ml 1N HCl aufgenommen, zweimal mit je 20 ml Essigester extrahiert, die vereinigten organischen Phasen mit ges. Kochsalzlösung gewaschen und über Natriumsulfat getrocknet. Dann wurde vom Lösungsmittel befreit und am Hochvakuum getrocknet. Man erhielt 0.20 g (67%) N-[4-(2-Chlor-6-trifluormethyl-pyridin-4-ylsulfamoyl)-phenyl]-acetamid als orange-gelbe Kristalle; MS(ISP): me/e = 394 ($C_{14}H_{12}ClF_3N_3O_3S^+$).

0.20 g (0.0005 Mol) N-[4-(2-Chlor-6-trifluormethyl-pyridin-4-ylsulfamoyl)-phenyl]-acetamid wurden in einem Gemisch aus 5 ml 1N NaOH und 5 ml Dioxan gelöst und 5 Std. am Rückfluss gekocht. Nach dem Abkühlen destillierte man das Dioxan ab, stellte man mit 0.1N HCl auf pH 6, extrahierte die wässrige Phase mit Essigester, wusch mit ges. Kochsalzlösung gegen und trocknete die org. Phase über $Na_2SO_4$. Anschliessend chromatographierte man auf Kieselgel mit Essigester/Hexan 1:4. Man erhielt 0.06 g (34%) 4-Amino-N-(2-chlor-6-trifluormethyl-pyridin-4-yl)-benzolsulfonamid als hellgelbe Kristalle; Smp.: 179-181°C.

0.052 g (0.00015 Mol) 4-Amino-N-(2-chlor-6-trifluormethyl-pyridin-4-yl)-benzolsulfonamid wurden in 30 ml 8M Methylamin in Ethanol in einem Autoklaven 80 Std. bei 135°C gerührt. Man liess das Methylamin verdampfen, löste den Rückstand in Ethanol, versetzte mit 2 g Kieselgel, engte ein und chromatographierte den Rückstand auf Kieselgel mit Essigester/Hexan 1:2. Man erhielt 0.036 g (70%) 4-Amino-N-(2-methylamino-6-trifluormethyl-pyridin-4-yl)-benzolsulfonamid als farblose Kristalle; Smp.: > 68°C (Zers.).

## Beispiel A

Es werden in üblicher Weise Tabletten folgender Zusammensetzung hergestellt:

| | mg/Tablette |
|---|---|
| Wirkstoff | 100 |
| Lactose pulv. | 95 |
| Maisstärke weiss | 35 |
| Polyvinylpyrrolidon | 8 |
| Na-carboxymethylstärke | 10 |
| Magnesiumstearat | 2 |
| Tablettengewicht | 250 |

## Patentansprüche

1. Verwendung von Verbindungen der allgemeinen Formel I

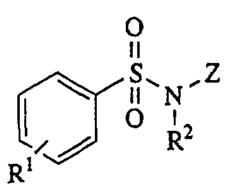

I

worin

Z eine substituierte Phenyl-, substituierte Pyridyl-, substituierte Pyrimidyl- und substituierte Indolylgruppe der Formeln a-e

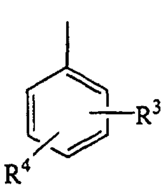
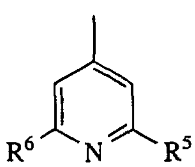
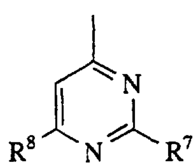
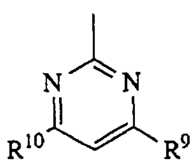
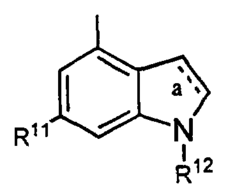

a , b , c , d und e

worin

$R^1$ Wasserstoff, Amino, $C_{1-7}$-Alkylamino, $C_{1-7}$-Dialkylamino, $C_{1-7}$-Alkyl, Halogen oder Trifluormethyl;

| | |
|---|---|
| $R^2$ | Wasserstoff oder $C_{1-7}$-Alkyl; |
| $R^3$ | Wasserstoff, Amino, $C_{1-7}$-Alkylamino, $C_{1-7}$-Dialkylamino, $C_{1-7}$-Alkyl, $CF_3$, $C_{1-7}$-Alkoxyoder Halogen; |
| $R^4$ | Amino, $C_{1-7}$-Alkylamino, $C_{1-7}$-Dialkylarnino, $C_{1-7}$-Alkyl, $C_{1-7}$-Alkoxy oder Halogen; |
| $R^5$ | Wasserstoff, $C_{1-7}$-Alkylamino, di- $C_{1-7}$-Alkylamino, $C_{1-7}$-Alkoxy oder Halogen; |
| $R^6$ | $C_{1-7}$-Alkyl, $C_{1-7}$-Alkylamino, di-$C_{1-7}$-Alkylamino, $C_{1-7}$-Alkoxy, Halogen oder $CF_3$; |
| $R^7$ | Amino, $C_{1-7}$-Alkylamino, di- $C_{1-7}$-Alkylamino, $C_{1-7}$-Alkoxy, $C_{1-7}$-Alkylsulfanyl, Mercapto, Pyrrolidin-1-yl oder Azetidin-1-yl; |
| $R^8$ | Amino, $C_{1-7}$-Alkylamino, di- $C_{1-7}$-Alkylamino,Benzylamino, $C_{1-7}$-Alkoxy, $C_{1-7}$-Alkylsulfanyl, Halogen, Pyrrolidin-1-yl oder Azetidin-1-yl; |
| $R^9$ und $R^{10}$ | unabhängig voneinander $C_{1-7}$-Alkoxy oder $C_{1-7}$-Alkylamino; |
| $R^{11}$ | Wasserstoff oder Halogen; |
| $R^{12}$ | Wasserstoff oder $C_{1-7}$-Alkyl, und |
| a | gegebenenfalls eine Doppelbindung bedeuten |

mit der Massgabe, dass $R^7$ und $R^8$ nicht gleichzeitig Methoxy bedeuten,
sowie deren pharmazeutisch annehmbare Salze zur Herstellung von Medikamenten zur Behandlung von zentralnervösen Störungen.

**2.** Verwendung gemäss Anspruch 1 zur Behandlung von Psychosen, Schizophrenie, manischen Depressionen, Depressionen, neurologischen Störungen, Gedächtnisstörungen, Parkinson'scher Krankheit, amyotropher Lateralsklerose, Alzheimer'scher Krankheit und Huntington'scher Krankheit.

**3.** Verwendung von Verbindungen der Formel Ia aus der Gruppe der Verbindungen der Formel I gemäss Ansprüchen 1 und 2,

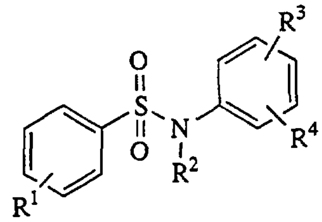

Ia

worin $R^1$, $R^2$, $R^3$ und $R^4$ die in Anspruch 1 angegebenen Bedeutungen haben.

**4.** Verwendung von Verbindungen der Formel Ib aus der Gruppe der Verbindungen der Formel I gemäss Ansprüchen 1 und 2,

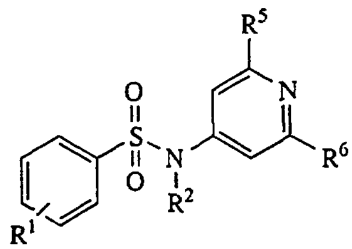

I b

worin $R^1$, $R^2$, $R^5$ und $R^6$ die in Anspruch 1 angegebenen Bedeutungen haben.

**5.** Verwendung von Verbindungen der Formeln Ic und Id aus der Gruppe der Verbindungen der Formel I gemäss Ansprüchen 1 und 2,

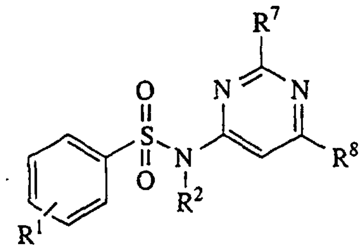

I c ,

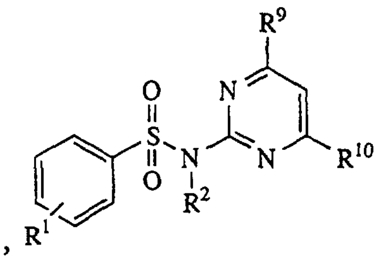

I d

worin $R^1$, $R^2$, $R^7$, $R^8$, $R^9$ und $R^{10}$ die in Anspruch 1 angegebenen Bedeutungen haben.

**6.** Verwendung von Verbindungen der Formel Ie aus der Gruppe der Verbindungen der Formel I gemäss Ansprüchen 1 und 2,

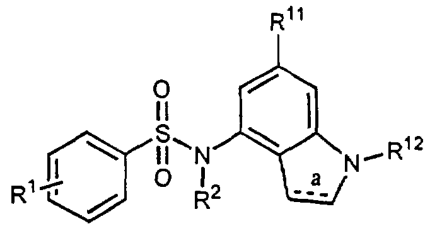

I e

worin $R^1$, $R^2$, $R^{11}$, $R^{12}$ und a die in Formel I angegebene Bedeutung haben.

**7.** Verwendung von

4-Amino-N-(2,6-bis-methylamino-pyrimidin-4-yl)-benzolsulfonamid;

4-Amino-N-(6-ethylamino-2-methylamino-pyrimidin-4-yl)-benzolsulfonamid;

4-Amino-N-(2-dimethylamino-6-methylamino-pyrimidin-4-yl)-benzolsulfonamid;

4-Amino-N-(2-dimethylamino-6-ethylamino-pyrimidin-4-yl)-benzolsulfonamid;

4-Amino-N-(2,6-bis-methylamino-pyrimidin-4-yl)-N-methyl-benzol-sulfonamid;

4-Amino-N-(2-azetidin-1-yl-6-methylamino-pyrimidin-4-yl)-benzolsulfonamid;

4-Amino-N-(2-azetidin-1-yl-6-ethylamino-pyrimidin-4-yl)-benzolsulfon-amid;

4-Amino-N-(6-methylamino-2-pyrrolidin-1-yl-pyrimidin-4-yl)-benzolsulfonamid;

4-Amino-N-(2-brom-6-methylamino-pyridin-4-yl)-benzolsulfonamid;

4-Amino-N-(2,6-bis-methylamino-pyridin-4-yl)-benzolsulfonamide;

4-Amino-N-(2-ethylamino-6-methylamino-pyridin-4-yl)-benzolsulfonamid und

4-Amino-N-(2-dimethylamino-6-methylamino-pyridin-4-yl)-benzolsulfonamid

4-Amino-N-(2,6-bis-ethylamino-pyridin-4-yl)-benzolsulfonamid;

N-(2,6-Bis-methylamino-pyrimidin-4-yl)-3-chlor-benzolsulfonamid;

N-(2,6-Bis-methylamino-pyrimidin-4-yl)-3-trifluormethyl-benzolsulfonamid;

4-Amino-N-(2-methyl-6-methylamino-pyridin-4-yl)-benzolsulfonamid;

4-Amino-N-(3,5-dimethoxy-phenyl)-benzolsulfonamid;

4-Amino-N-(3,5-dichlor-phenyl)-benzolsulfonamid;

4-Amino-N-(3,5-dibrom-phenyl)-benzolsulfonamid und

4-Amino-N-(1H-indol-4-yl)-benzolsulfonamid

gemäss Ansprüchen 1 - 6.

**8.** Verbindungen der Formeln $Ia_1$ und $Ia_2$ aus der Gruppe der Verbindungen der Formel Ia aus Anspruch 3,

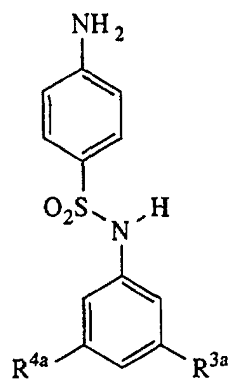

$Ia_1$ ,

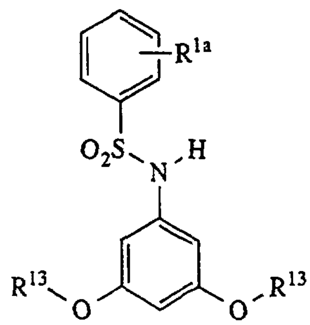

$Ia_2$

worin

$R^{1a}$ 3-Trifluormethyl, 3-Halogen oder 4-Halogen;
$R^{3a}$ Wasserstoff, Halogen, $C_{1-7}$-Alkoxy, Amino oder $C_{1-7}$-Alkylamino;
$R^{4a}$ Amino oder $C_{1-7}$-Alkylamino und
$R^{13}$ $C_{1-7}$-Alkyl bedeuten,

mit der Einschränkung, dass, wenn $R^{4a}$ Amino bedeutet, $R^{3a}$ von Wasserstoff verschieden ist, sowie deren pharmazeutisch annehmbare Salze.

**9.** Verbindungen der Formel $Ia_1$ gemäss Anspruch 8, worin

$R^{3a}$ Wasserstoff, Amino oder Methylamino;
$R^{4a}$ Amino oder Methylamino bedeuten;

sowie deren pharmazeutisch annehmbare Salze.

**10.** Verbindung der Formel Ia2 gemäss Anspruch 8, worin

$R^{1a}$ 3-Trifluor-methyl;
$R^{13}$ Methyl bedeuten;

sowie deren pharmazeutisch annehmbare Salze.

**11.** Verbindungen der Formeln $Ib_1$ und $Ib\ _2$ aus der Gruppe der Verbindungen der Formel Ib aus Anspruch 4,

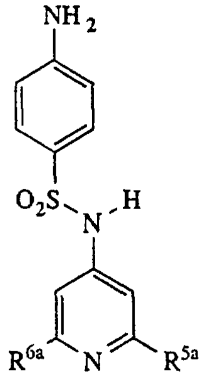

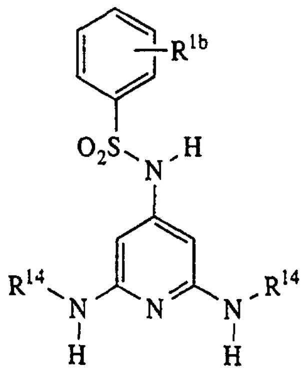

$Ib_1$ , $Ib_2$

worin

$R^{1b}$ 4-Halogen, 3-Halogen oder 3-Trifluormethyl;
$R^{5a}$ Wasserstoff, $C_{1-7}$-Alkylamino, di- $C_{1-7}$-Alkylamino oder Halogen;
$R^{6a}$ $C_{1-7}$-Alkyl, $CF_3$, $C_{1-7}$-Alkylamino, di- $C_{1-7}$-Alkylamino, oder Halogen und
$R^{14}$ $C_{1-7}$-Alkyl bedeuten,

mit der Einschränkung, dass, wenn $R^{6a}$ Halogen bedeutet, $R^{5a}$ von Wasserstoff verschieden ist, sowie deren pharmazeutisch annehmbare Salze.

**12.** Verbindungen der Formeln $Ib_1$ gemäss Anspruch 11, worin

$R^{5a}$ Wasserstoff, Methylamino, Ethylamino, di-Methylamino, Chlor oder Brom und
$R^{6a}$ Methyl, Methylamino, Ethylamino, di-Methylamino oder Brom bedeuten,

mit der Einschränkung, dass wenn $R^{6a}$ Brom bedeutet, $R^{5a}$ von Wasserstoff verschieden ist, sowie deren pharmazeutisch annehmbare Salze.

**13.** Verbindungen der Formeln $Ib_2$ gemäss Anspruch 11, worin

$R^{1b}$ 4-Chlor, 3-Chlor oder 3-Trifluor-methyl;
$R^{14}$ Methyl bedeuten;

sowie deren pharmazeutisch annehmbare Salze.

**14.** Verbindungen der Formeln $Ic_1$ und $Ic_2$ aus der Gruppe der Verbindungen der Formel Ic aus Anspruch 5,

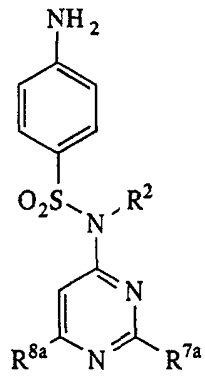

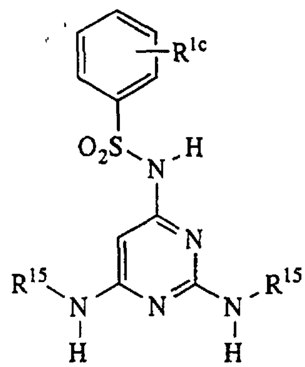

Ic$_1$ , Ic$_2$

worin

- $R^{1c}$ Wasserstoff, 4-Halogen, 4-niederes Alkyl, 3-Halogen, 3- Trifluormethyl;
- $R^2$ Wasserstoff oder $C_{1-7}$-Alkyl;
- $R^{7a}$ Amino, $C_{1-7}$-Alkylamino, di- $C_{1-7}$-Alkylamino, Mercapto, Pyrrolidin-1-yl oder Azetidin-1-yl;
- $R^{8a}$ Amino, $C_{1-7}$-Alkylamino, di- $C_{1-7}$-Alkylamino,Benzylamino, $C_{1-7}$-Alkoxy, Pyrrolidin-1-yl oder Azetidin-1-yl und
- $R^{15}$ $C_{1-7}$-Alkyl bedeuten,

mit der Einschränkung, dass, wenn $R^{7a}$ di- $C_{1-7}$-Alkylamino bedeutet, $R^{8a}$ von $C_{1-7}$-Alkoxy und von di- $C_{1-7}$-Alkylamino verschieden ist, sowie deren pharmazeutisch annehmbare Salze.

**15.** Verbindungen der Formel Ic$_1$ gemäss Anspruch 14, worin

- $R^2$ Wasserstoff oder Methyl;
- $R^{7a}$ Amino, Methylamino, Ethylamino, Propylamino, iso-Propylamino, Dimethylamino, Mercapto, Pyrrolidin-1-yl oder Azetidin-1-yl;
- $R^{8a}$ Amino, Methylamino, Ethylamino, Propylamino, iso-Propylamino, Dimethylamino, Benzylamino, Methoxy, Pyrrolidin-1-yl oder Azetidin-1-yl bedeuten,

mit der Einschränkung, dass, wenn $R^{7a}$ Dimethylamino bedeutet, $R^{8a}$ von Dimethylamino und Methoxy verschieden ist, sowie deren pharmazeutisch annehmbare Salze.

**16.** Verbindungen der Formel Ic$_2$ gemäss Anspruch 14, worin

- $R^{1c}$ Wasserstoff, 4-Fluor, 4-Chlor, 4-Methyl, 4-t. Butyl, 3-Chlor, 3-Trifluormethyl und
- $R^{15}$ Methyl bedeuten;

sowie deren pharmazeutisch annehmbare Salze.

**17.** Verbindungen der Formeln Id$_1$ und Id$_2$ aus der Gruppe der Verbindungen der Formel Id aus Anspruch 5,

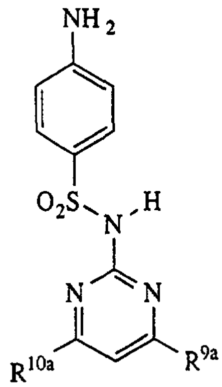

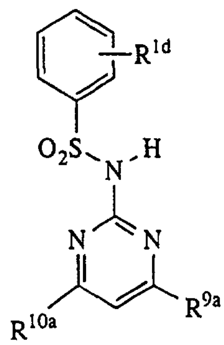

Id$_1$ , Id$_2$

worin

| | |
|---|---|
| $R^{1d}$ | 3-Trifluormethyl, 4-Trifluormethyl, 3-Halogen oder 4-Halogen und |
| $R^{9a}$ und $R^{10a}$ | $C_{1-7}$-Alkylamino bedeuten, |

sowie deren pharmazeutisch annehmbare Salze.

**18.** Verbindungen der Formel Ie aus Anspruch 6,

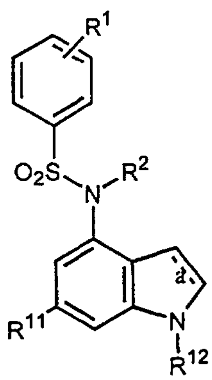

Ie

worin $R^1$, $R^2$, $R^{11}$, $R^{12}$ und a die in Formel I angegebene Bedeutung haben.

**19.** Arzneimittel, insbesondere zur Behandlung und/oder Verhütung von zentralnervösen Störungen, enthaltend eine oder mehrere Verbindungen nach einem der Ansprüche 8 - 18 und mindestens ein therapeutisch inertes Excipiens.

**20.** Verfahren zur Herstellung von Verbindungen der Formel I gemäss Ansprüchen 8 - 18 und ihrer Salze aus einer Verbindung der Formel II,

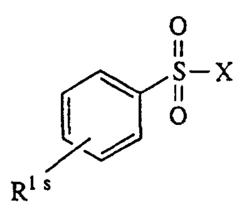

II

worin

$R^{1s}$ die für $R^1$ angegebene Bedeutung hat oder geschütztes Amino und
X Halogen oder -NHY bedeuten, und Y für ein Alkalimetall, beispielsweise Natrium oder Kalium, steht,

**dadurch gekennzeichnet, dass** man eine Verbindung der Formel II, in der X Halogen bedeutet,

a) mit einer Verbindung der Formel

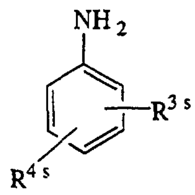

III a

worin

$R^{3s}$ Wasserstoff, $C_{1-7}$-Alkoxy, Halogen, geschütztes Amino oder geschütztes $C_{1-7}$-Alkylamino und
$R^{4s}$ geschütztes Amino oder geschütztes $C_{1-7}$-Alkylamino bedeuten, umsetzt und bedeuten, umsetzt und

die Aminoschutzgruppen abspaltet; oder

b) mit einer Verbindung der Formel

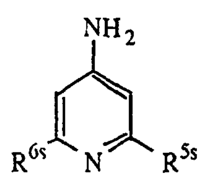

III b

worin

$R^{5s}$ Halogen und
$R^{6s}$ Halogen, $C_{1-7}$-Alkyl oder $CF_3$ bedeuten, umsetzt und das Reaktionsprodukt gegebenenfalls mit $C_{1-7}$-Alkylamin oder di- $C_{1-7}$-Alkylamin behandelt und die Aminoschutzgruppe abspaltet; oder

c) mit einer Verbindung der Formel

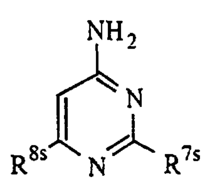

III c

worin

$R^{7s}$ Mercapto und
$R^{8s}$ Amino, $C_{1-7}$-Alkylamino, di- $C_{1-7}$-Alkylamino, Benzylamino, $C_{1-7}$-Alkoxy, Pyrrolidin-1-yl oder Azetidin-1-yl bedeuten, umsetzt und gegebenenfalls die Aminoschutzgruppe abspaltet, oder

d) eine Verbindung der Formel II, in der X -NHY bedeutet, zunächst mit einer Verbindung der Formel

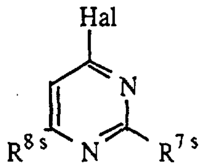

IIId

worin

$R^{7s}$ Amino, $C_{1-7}$-Alkylamino, di- $C_{1-7}$-Alkylamino, Pyrrolidin-1-yl oder Azetidin-1-yl und
$R^{8s}$ Halogen bedeuten, umsetzt, und dann das Reaktionsprodukt gegebenenfalls mit einem $C_{1-7}$-Alkylamin, di- $C_{1-7}$-Alkylamin,Azetidin, Pyrrolidin oder einem Alkoholat behandelt; oder

e) eine Verbindung der Formel II, in der X Halogen bedeutet, mit einerVerbindung der Formel

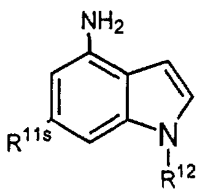

IIIe

worin
$R^{11s}$ Wasserstoff oder Halogen und $R^{12}$ Wasserstoff oder $C_{1-7}$-Alkyl bedeuten, umsetzt und anschliessend gegebenenfalls zu eine Verbindung der Formel Ie reduziert, worin a keine Doppelbindung bedeutet, oder dadurch, dass man

f) eine Verbindung der Formel

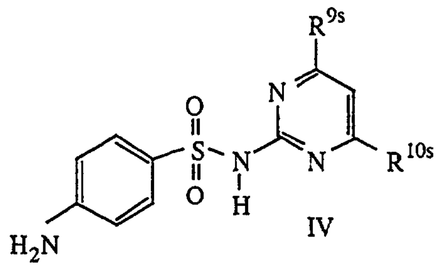

IV

worin
$R^{9s}$ und $R^{10s}$ $C_{1-7}$-Alkoxy bedeuten, mit einem $C_{1-7}$-Alkylamin umsetzt, oder

g) ein Sulfadimethoxin der Formel V

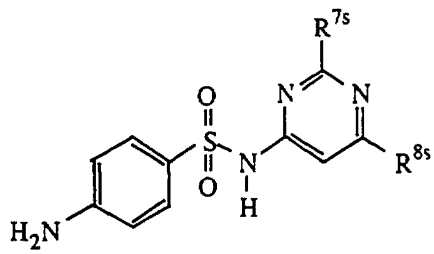

V

worin

$R^{7s}$ und $R^{8s}$ $C_{1-7}$-Alkoxy bedeuten, mit einem $C_{1-7}$-Alkylamin umsetzt und

h) erwünschtenfalls eine Verbindung der allgemeinen Formel I in ein pharmazeutisch annehmbares Salz überführt.

**21.** Verbindungen nach einem der Ansprüche 8 - 18 zur Anwendung als therapeutische Wirkstoffe, im Speziellen zur Bekämpfung oder Verhütung von zentralnervösen Störungen.

**22.** Verwendung von Verbindungen nach einem der Ansprüche 8 - 18 zur Herstellung eines Medikamentes zur Bekämpfung oder Verhütung von zentralnervösen Störungen.

**23.** Verwendung gemäss Anspruch 22 bei der Bekämpfung oder Verhütung von zentralnervösen Störungen wie Psychosen, Schizophrenie, manischen Depressionen, Depressionen, neurologischen Störungen, Gedächtnisstörungen, Parkinson'scher Krankheit, amyotropher Lateralsklerose, Alzheimer'scher Krankheit und Huntington'scher Krankheit.

## Claims

**1.** The use of compounds of general formula I

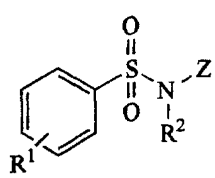

I

wherein

Z signifies a substituted phenyl, substituted pyridyl, substituted pyrimidyl and substituted indolyl group of formulae a-e

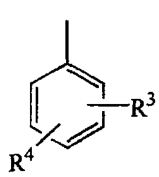

a ,

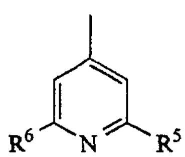

b ,

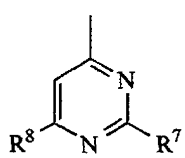

c ,

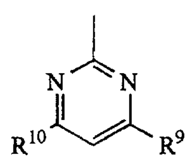

d and

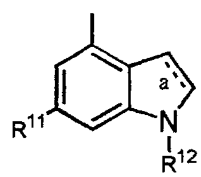

e

wherein

| | |
|---|---|
| $R^1$ | signifies hydrogen, amino, $C_{1-7}$-alkylamino, $C_{1-7}$-dialkylamino, $C_{1-7}$-alkyl, halogen or trifluoromethyl; |
| $R^2$ | signifies hydrogen or $C_{1-7}$-alkyl; |
| $R^3$ | signifies hydrogen, amino, $C_{1-7}$-alkylamino, $C_{1-7}$-dialkylamino, $C_{1-7}$-alkyl, $CF_3$, $C_{1-7}$-alkoxy or halogen; |
| $R^4$ | signifies amino, $C_{1-7}$-alkylamino, $C_{1-7}$-dialkylamino, $C_{1-7}$-alkyl, $C_{1-7}$-alkoxy or halogen; |
| $R^5$ | signifies hydrogen, $C_{1-7}$-alkylamino, di-$C_{1-7}$-alkylamino, $C_{1-7}$-alkoxy or halogen; |
| $R^6$ | signifies $C_{1-7}$-alkyl, $C_{1-7}$-alkylamino, di-$C_{1-7}$-alkylamino, $C_{1-7}$-alkoxy, halogen or $CF_3$; |
| $R^7$ | signifies amino, $C_{1-7}$-alkylamino, di-$C_{1-7}$-alkylamino, $C_{1-7}$-alkoxy, $C_{1-7}$-alkylsulphanyl, mercapto, pyrrolidin-1-yl or azetidin-1-yl; |
| $R^8$ | signifies amino, $C_{1-7}$-alkylamino, di-$C_{1-7}$-alkylamino, benzylamino, $C_{1-7}$-alkoxy, $C_{1-7}$-alkylsulphanyl, halogen, pyrrolidin-1-yl or azetidin-1-yl; |
| $R^9$ and $R^{10}$ | each independently signify $C_{1-7}$-alkoxy or $C_{1-7}$-alkylamino; |
| $R^{11}$ | signifies hydrogen or halogen; |
| $R^{12}$ | signifies hydrogen or $C_{1-7}$-alkyl and |
| a | signifies an optional double bond, |

with the proviso that $R^7$ and $R^8$ do not simultaneously signify methoxy, as well as their pharmaceutically acceptable salts for the production of medicaments for the treatment of central nervous disorders.

2. The use according to claim 1 for the treatment of phychoses, schizophrenia, manic depressions, depressions, neurological disorders, memory disorders, Parkinson's disease, amyotrophic lateral sclerosis, Alzheimer's disease and Huntington's disease.

3. The use in accordance with claims 1 and 2 of compounds of formula Ia from the group of compounds of formula I

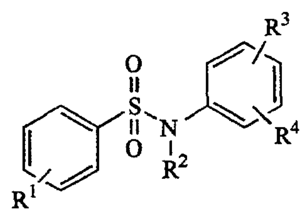

Ia

wherein $R^1$, $R^2$, $R^3$ and $R^4$ have the significances given in claim 1.

4. The use according to claim 1 or 2 of compounds of formula Ib from the group of compounds of formula I

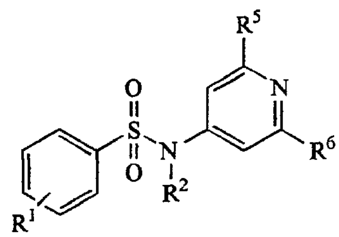

I b

wherein $R^1$, $R^2$, $R^5$ and $R^6$ have the significances given in claim 1.

5. The use according to claim 1 or 2 of compounds of formulae Ic and Id from the group of compounds of formula I

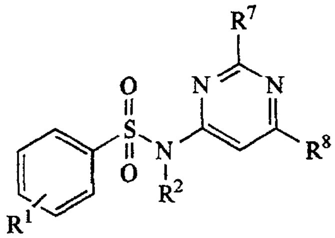 Ic , 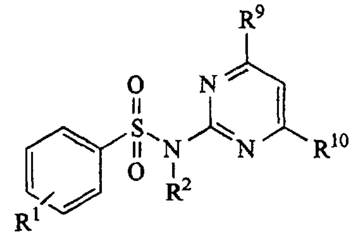 Id

wherein $R^1$, $R^2$, $R^7$, $R^8$, $R^9$ and $R^{10}$ have the significances given in claim 1.

**6.** The use according to claim 1 or 2 of compounds of formula Ie from the group of formula I

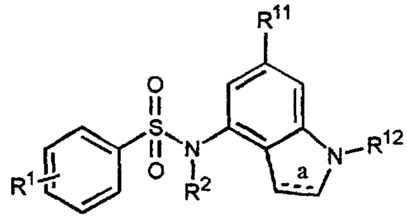 Ie

wherein $R^1$, $R^2$, $R^{11}$, $R^{12}$ and a have the significance given in claim 1.

**7.** The use of

4-amino-N-(2,6-bis-methylamino-pyrimidin-4-yl)-benzenesulphonamide;
4-amino-N-(6-ethylamino-2-methylamino-pyrimidin-4-yl)-benzenesulphonamide;
4-amino-N-(2-dimethylamino-6-methylamino-pyrimidin-4-yl)-benzenesulphonamide;
4-amino-N-(2-dimethylamino-6-ethylamino-pyrimidin-4-yl)-benzenesulphonamide;
4-amino-N-(2,6-bis-methylamino-pyrimidin-4-yl)-N-methyl-benzenesulphonamide;
4-amino-N-(2-azetidin-1-yl-6-methylamino-pyrimidin-4-yl) -benzenesulphonamide;
4-amino-N-(2-azetidin-1-yl-6-ethylamino-pyrimidin-4-yl)-benzenesulphonamide;
4-amino-N-(6-methylamino-2-pyrrolidin-1-yl-pyrimidin-4-yl) -benzenesulphonamide;
4-amino-N-(2-bromo-6-methylamino-pyridin-4-yl) -benzenesulphonamide;
4-amino-N-(2,6-bis-methylamino-pyridin-4-yl)-benzenesulphonamide;
4-amino-N-(2-ethylamino-6-methylamino-pyridin-4-yl) -benzenesulphonamide;
4-amino-N-(2-dimethylamino-6-methylamino-pyridin-4-yl)-benzenesulphonamide
4-amino-N-(2,6-bis-ethylamino-pyridin-4-yl)-benzenesulphonamide;
N-(2,6-bis-methylamino-pyrimidin-4-yl)-3-chloro-benzenesulphonamide;
N-(2,6-bis-methylamino-pyrimidin-4-yl)-3-trifluoromethyl-benzenesulphonamide;
4-amino-N-(2-methyl-6-methylamino-pyridin-4-yl)-benzenesulphonamide;
4-amino-N-(3,5-dimethoxy-phenyl)-benzenesulphonamide;
4-amino-N- (3,5-dichloro-phenyl) -benzenesulphonamide;
4-amino-N-(3,5-dibrom-phenyl)-benzenesulphonamide and
4-amino-N-(1H-indol-4-yl)-benzenesulphonamide

in accordance with claims 1-6.

**8.** Compounds of formulae $Ia_1$ and $Ia_2$ from the group of compounds of formula Ia from claim 3

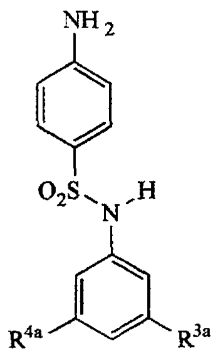 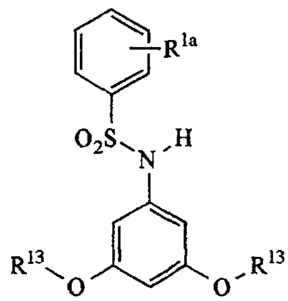

$Ia_1$ $Ia_2$

wherein

$R^{1a}$ signifies 3-trifluoromethyl, 3-halo or 4-halo;
$R^{3a}$ signifies hydrogen, halogen, $C_{1-7}$-alkoxy, amino or $C_{1-7}$-alkylamino;
$R^{4a}$ signifies amino or $C_{1-7}$-alkylamino and
$R^{13}$ signifies $C_{1-7}$-alkyl,

with the proviso that $R^{3a}$ is different from hydrogen when $R^{4a}$ signifies amino, as well as their pharmaceutically acceptable salts.

**9.** Compounds of formula $Ia_1$ according to claim 8, wherein

$R^{3a}$ signifies hydrogen, amino or methylamino;
$R^{4a}$ signifies amino or methylamino;

as well as their pharmaceutically acceptable salts.

**10.** Compounds of formula $Ia_2$ according to claim 8, wherein

$R^{1a}$ signifies 3-trifluoro-methyl;
$R^{13}$ signifies methyl;

as well as their pharmaceutically acceptable salts.

**11.** Compounds of formulae $Ib_1$ and $Ib_2$ from the group of compounds of formula Ib from claim 4

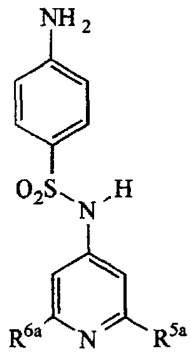

Ib₁ ,

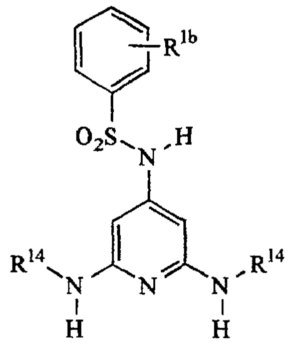

Ib₂

wherein

$R^{1b}$ signifies 4-halo, 3-halo or 3-trifluoromethyl;
$R^{5a}$ signifies hydrogen, $C_{1-7}$-alkylamino, di-$C_{1-7}$-alkylamino or halogen;
$R^{6a}$ signifies $C_{1-7}$-alkyl, $CF_3$, $C_{1-7}$-alkylamino, di-$C_{1-7}$-alkylamino, or halogen and
$R^{14}$ signifies $C_{1-7}$-alkyl;

with the proviso that $R^{5a}$ is different from hydrogen when $R^{6a}$ signifies halogen, as well as their pharmaceutically acceptable salts.

**12.** Compounds of formulae $Ib_1$ according to claim 11, wherein

$R^{5a}$ signifies hydrogen, methylamino, ethylamino, di-methylamino, chlorine or bromine and
$R^{6a}$ signifies methyl, methylamino, ethylamino, di-methylamino or bromine,

with the proviso that $R^{5a}$ is different from hydrogen when $R^{6a}$ signifies bromine, as well as their pharmaceutically acceptable salts.

**13.** Compounds of formulae $Ib_2$ according to claim 11, wherein

$R^{1b}$ signifies 4-chloro, 3-chloro or 3-trifluoro-methyl;
$R^{14}$ signifies methyl,

as well as their pharmaceutically acceptable salts.

**14.** Compounds of formulae $Ic_1$ and $Ic_2$ from the group of compounds of formula Ic from claim 5

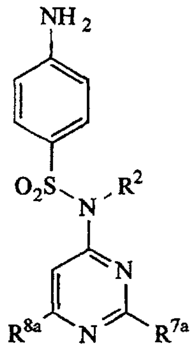 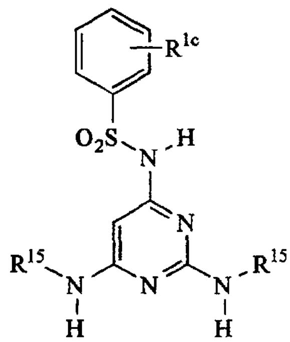

Ic$_1$ , Ic$_2$

wherein

$R^{1c}$ signifies hydrogen, 4-halo, 4-lower alkyl, 3-halo, 3-trifluoromethyl;
$R^2$ signifies hydrogen or $C_{1-7}$-alkyl;
$R^{7a}$ signifies amino, $C_{1-7}$-alkylamino, di-$C_{1-7}$-alkylamino, mercapto, pyrrolidin-1-yl or azetidin-1-yl;
$R^{8a}$ signifies amino, $C_{1-7}$-alkylamino, di-$C_{1-7}$-alkylamino, benzylamino, $C_{1-7}$-alkoxy, pyrrolidin-1-yl or azetidin-1-yl and
$R^{15}$ signifies $C_{1-7}$-alkyl,

with the proviso that $R^{8a}$ is different from $C_{1-7}$-alkoxy and from di-$C_{1-7}$-alkylamino when $R^{7a}$ signifies di-$C_{1-7}$-alkylamino,
as well as their pharmaceutically acceptable salts.

**15.** Compounds of formula Ic$_1$ according to claim 14, wherein

$R^2$ signifies hydrogen or methyl;
$R^{7a}$ signifies amino, methylamino, ethylamino, propylamino, isopropylamino, dimethylamino, mercapto, pyrrolidin-1-yl or azetidin-1-yl;
$R^{8a}$ signifies amino, methylamino, ethylamino, propylamino, isopropylamino, dimethylamino, benzylamino, methoxy, pyrrolidin-1-yl or azetidin-1-yl,

with the proviso that $R^{8a}$ is different from dimethylamino and methoxy when $R^{7a}$ signifies dimethylamino, as well as their pharmaceutically acceptable salts.

**16.** Compounds of formula Ic$_2$ according to claim 14, wherein

$R^{1c}$ signifies hydrogen, 4-fluoro, 4-chloro, 4-methyl, 4-t. butyl, 3-chloro, 3-trifluoromethyl and
$R^{15}$ signifies methyl;

as well as their pharmaceutically acceptable salts.

**17.** Compounds of formulae Id$_1$ and Id$_2$ from the group of compounds of formula Id from claim 5

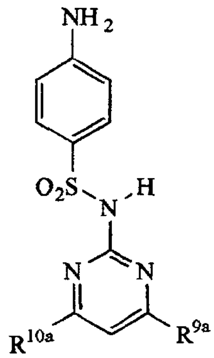
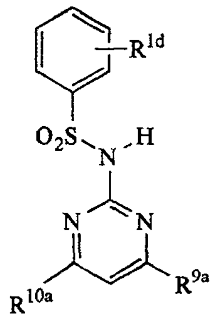

Id 1 , Id 2

wherein

$R^{1d}$ signifies 3-trifluoromethyl, 4-trifluoromethyl, 3-halo or 4-halo and
$R^{9a}$ and $R^{10a}$ signify $C_{1-7}$-alkylamino,

as well as their pharmaceutically acceptable salts.

**18.** Compounds of formula Ie from claim 6

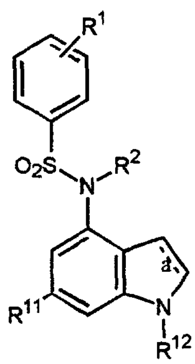
I e

wherein $R^1$, $R^2$, $R^{11}$, $R^{12}$ and a have the significance given in claim 1.

**19.** A medicament, especially for the treatment and/or prevention of central nervous disorders, containing one or more compounds according to any one of claims 8-18 and at least one therapeutically inert excipient.

**20.** A process for the manufacture of the compounds of formula I according to any one of claims 8-18 and their salts from a compound of formula II

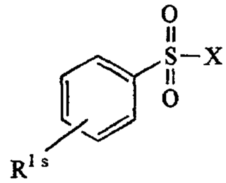
II

wherein

$R^{1s}$ has the significance given for $R^1$ or signifies protected amino and

X signifies halogen or -NHY, and Y stands for an alkali metal, for example sodium or potassium,

**characterized by** reacting a compound of formula II in which X signifies halogen

a) with a compound of the formula

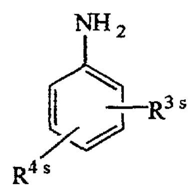

III a

wherein

$R^{3s}$ signifies hydrogen, $C_{1-7}$-alkoxy, halogen, protected amino or protected $C_{1-7}$-alkylamino and
$R^{4s}$ signifies protected amino or protected $C_{1-7}$-alkylamino,

and cleaving of the amino protecting groups; or

b) with a compound of the formula

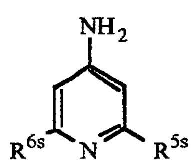

III b

wherein

$R^{5s}$ signifies halogen and
$R^{6s}$ signifies halogen, $C_{1-7}$-alkyl or $CF_3$,

and, if desired, treating the reaction product with a $C_{1-7}$-alkylamine or di-$C_{1-7}$-alkylamine and cleaving off the amino protecting group; or

c) with a compound of the formula

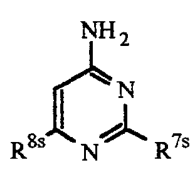

III c

wherein

$R^{7s}$ signifies mercapto and
$R^{8s}$ signifies amino, $C_{1-7}$-alkylamino, di-$C_{1-7}$-alkylamino, benzylamino, $C_{1-7}$-alkoxy, pyrrolidin-1-yl or azetidin-1-yl,

and, if necessary, cleaving off the amino protecting group; or

d) reacting a compound of formula II in which X signifies -NHY firstly with a compound of the formula

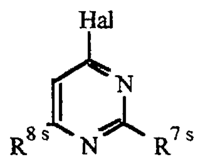

IIId

wherein

$R^{7s}$ signifies amino, $C_{1-7}$-alkylamino, di-$C_{1-7}$-alkylamino, pyrrolidin-1-yl or azetidin-1-yl and
$R^{8s}$ signifies halogen,

and then, if desired, treating the reaction product with a $C_{1-7}$-alkylamine, di-$C_{1-7}$-alkylamine, azetidine, pyrrolidine or an alcoholate; or

e) reacting a compound of formula II in which X signifies halogen with a compound of the formula

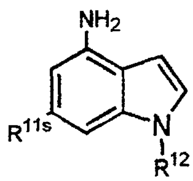

IIIe

wherein

$R^{11s}$ signifies hydrogen or halogen and $R^{12}$ signifies hydrogen or $C_{1-7}$-alkyl,

and subsequently, if desired, reducing to a compound of formula Ie in which a does not signify a double bond; or

f) reacting a compound of the formula

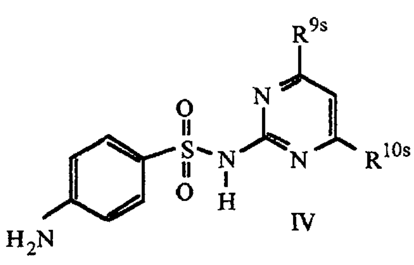

IV

wherein

$R^{9s}$ and $R^{10s}$ signify $C_{1-7}$-alkoxy,

with a $C_{1-7}$-alkylamine; or

g) reacting a sulfadimethoxine of the formula

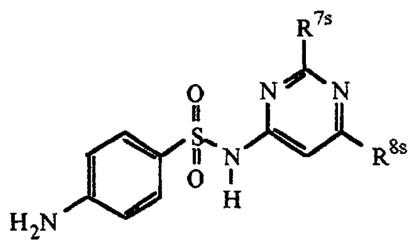

V

wherein

$R^{7s}$ and $R^{8s}$ signify $C_{1-7}$-alkoxy,

with a $C_{1-7}$-alkylamine, and

h) if desired, converting a compound of general formula I into a pharmaceutically acceptable salt.

**21.** Compounds according to any one of claims 8-18 for use as therapeutically active substances, especially for the control or prevention of central nervous disorders.

**22.** The use of compounds according to any one of claims 8-18 for the production of a medicament for the control or prevention of central nervous disorders.

**23.** The use according to claim 22 in the control of prevention of central nervous disorders such as psychoses, schizophrenia, manic depressions, depressions, neurological disorders, memory disorders, Parkinson's disease, amyotrophic lateral sclerosis, Alzheimer's disease and Huntington's disease.

**Revendications**

**1.** Utilisation de composés de formule générale I

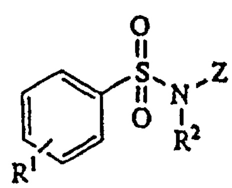

I

dans laquelle

Z représente un groupe phényle substitué, pyridyle substitué, pyrimidyle substitué ou indolyle substitué de formules a-e

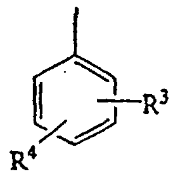
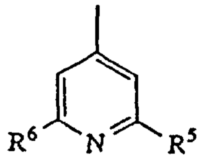
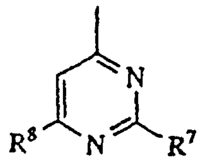
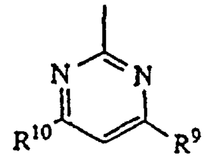
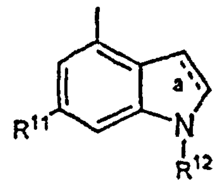

a , b , c , d et e

dans lesquelles

| | |
|---|---|
| $R^1$ | représente un atome d'hydrogène ou d'halogène, ou un groupe amino, alkyl($C_{1-7}$)amino, dialkyl ($C_{1-7}$)amino, alkyle en $C_{1-7}$ ou trifluorométhyle ; |
| $R^2$ | représente un atome d'hydrogène ou un groupe alkyle en $C_{1-7}$ ; |
| $R^3$ | représente un atome d'hydrogène ou d'halogène, ou un groupe amino, alkyl($C_{1-7}$)amino, dialkyl ($C_{1-7}$)amino, alkyle en $C_{1-7}$, $CF_3$ ou alcoxy en $C_{1-7}$ ; |
| $R^4$ | représente un atome d'halogène, ou un groupe amino, alkyl($C_{1-7}$)amino, dialkyl($C_{1-7}$)amino, alkyle en $C_{1-7}$ ou alcoxy en $C_{1-7}$ ; |
| $R^5$ | représente un atome d'hydrogène ou d'halogène, ou un groupe alkyl($C_{1-7}$)amino, dialkyl($C_{1-7}$) amino ou alcoxy en $C_{1-7}$ ; |
| $R^6$ | représente un atome d'halogène ou un groupe alkyle en $C_{1-7}$, alkyl($C_{1-7}$)amino, dialkyl($C_{1-7}$)amino, alcoxy en $C_{1-7}$ ou $CF_3$ ; |
| $R^7$ | représente un groupe amino, alkyl($C_{1-7}$)amino, dialkyl($C_{1-7}$)amino, alcoxy en $C_{1-7}$, alkyl($C_{1-7}$) sulfanyle, mercapto, pyrrolidin-1-yle ou azétidin-1-yle ; |
| $R^8$ | représente un atome d'halogène ou un groupe amino, alkyl($C_{1-7}$)amino, dialkyl($C_{1-7}$)amino, benzylamino, alcoxy en $C_{1-7}$, alkyl($C_{1-7}$)sulfanyle, pyrrolidin-1-yle ou azétidin-1-yle ; |
| $R^9$ et $R^{10}$ | représentent, indépendamment l'un de l'autre, un groupe alcoxy en $C_{1-7}$ ou alkyl($C_{1-7}$)amino ; |
| $R^{11}$ | représente un atome d'hydrogène ou d'halogène ; |
| $R^{12}$ | représente un atome d'hydrogène ou un groupe alkyle en $C_{1-7}$ et |
| a | représente éventuellement une double liaison |

étant entendu que $R^7$ et $R^8$ ne représentent pas simultanément le groupe méthoxy,
ainsi que de leurs sels pharmaceutiquement acceptables, pour la fabrication de médicaments destinés au traitement de troubles du système nerveux central.

**2.** Utilisation selon la revendication 1, pour le traitement de psychoses, de la schizophrénie, de psychoses maniacodépressives, de dépressions, de troubles neurologiques, de troubles de la mémoire, de la maladie de Parkinson, de la sclérose latérale amyotrophique, de la maladie d'Alzheimer et de la chorée de Huntington.

**3.** Utilisation de composés de formule la choisis dans le groupe des composés de formule I selon les revendications 1 et 2,

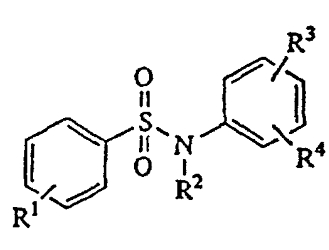

Ia

dans laquelle $R^1$, $R^2$, $R^3$ et $R^4$ ont les significations données dans la revendication 1.

**4.** Utilisation de composés de formule Ib choisis dans le groupe des composés de formule I selon les revendications 1 et 2,

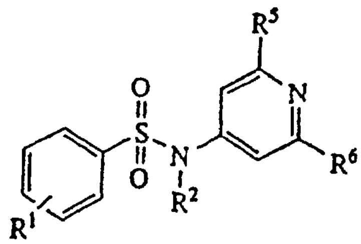

I b

dans laquelle $R^1$, $R^2$, $R^5$ et $R^6$ ont les significations données dans la revendication 1.

**5.** Utilisation de composés de formules Ic et Id choisis dans le groupe des composés de formule I selon les revendications 1 et 2,

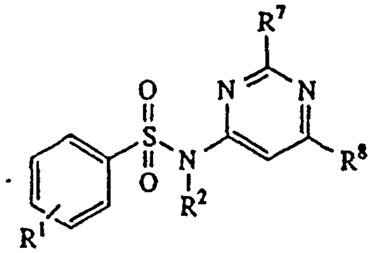

I c

,

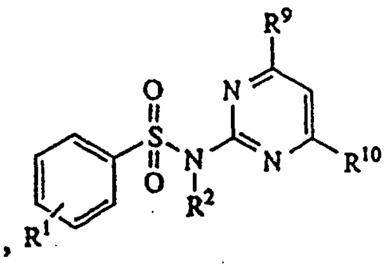

I d

formules dans lesquelles $R^1$, $R^2$, $R^7$, $R^8$, $R^9$ et $R^{10}$ ont les significations données dans la revendication 1.

**6.** Utilisation de composés de formule la choisis dans le groupe des composés de formule I selon les revendications 1 et 2,

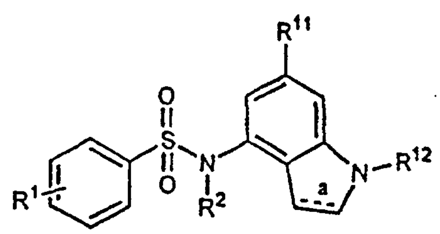

I e

dans laquelle $R^1$, $R^2$, $R^{11}$ et $R^{12}$ et a ont les significations données pour la formule I.

**7.** Utilisation du

4-amino-N-(2,6-bis-méthylamino-pyrimidin-4-yl)benzènesulfonamide,
4-amino-N-(6-éthylamino-2-méthylamino-pyrimidin-4-yl)benzènesulfonamide,
4-amino-N-(2-diméthylamino-6-méthylamino-pyrimidin-4-yl)benzènesulfonamide,
4-amino-N-(2-diméthylamino-6-éthylamino-pyrimidin-4-yl)benzènesulfonamide,
4-amino-N-(2,6-bis-méthylamino-pyrimidin-4-yl)-N-méthyl-benzènesulfonamide,
4-amino-N-(2-azétidin-1-yl-6-méthylamino-pyrimidin-4-yl)-benzènesulfonamide,
4-amino-N-(2-azétidin-1-yl-6-éthylamino-pyrimidin-4-yl)-benzènesulfonamide,
4-amino-N-(6-méthylamino-2-pyrrolidin-1-yl-pyrimidin-4-yl)-benzènesulfonamide
4-amino-N-(2-bromo-6-méthylamino-pyridin-4-yl)-benzènesulfonamide,
4-amino-N-(2,6-bis-méthylamino-pyridin-4-yl)-benzènesulfonamide;
4-amino-N-(2-éthylamino-6-méthylamino-pyridin-4-yl)-benzènesulfonamide et
4-amino-N-(2-diméthylamino-6-méthylamino-pyridin-4-yl)-benzènesulfonamide,

4-amino-N-(2,6-bis-éthylamino-pyridin-4-yl)-benzènesulfonamide,
N-(2,6-bis-méthylamino-pyrimidin-4-yl)-3-chlorobenzènesulfonamide,
N-(2,6-bis-méthylamino-pyrimidin-4-yl)-3-trifluorméthyl-benzènesulfonamide,
4-amino-N-(2-méthyl-6-méthylamino-pyridin-4-yl)-benzènesulfonamide;
4-amino-N-(3,5-diméthoxy-phényl)-benzènesulfonamide,
4-amino-N-(3,5-dichlorophényl)-benzènesulfonamide,
4-amino-N-(3,5-dibromophényl)-benzènesulfonamide et
4-amino-N-(1H-indol-4-yl)-benzènesulfonamide,

selon les revendications 1 à 6.

**8.** Composés de formules $Ia_1$ et $Ia_2$ choisis dans le groupe des composés de formule Ia de la revendication 3,

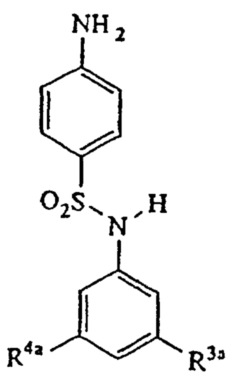

$Ia_1$ ,

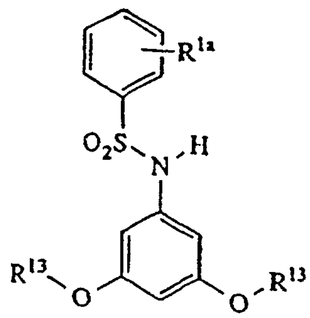

$Ia_2$

formules dans lesquelles

$R^{1a}$ représente un groupe 3-trifluorométhyle, 3-halogéno ou 4-halogéno ;
$R^{3a}$ représente un atome d'hydrogène ou d'halogène, ou un groupe alcoxy en $C_{1-7}$, amino ou alkyl($C_{1-7}$)amino ;
$R^{4a}$ représente un groupe amino ou alkyl($C_{1-7}$)amino et
$R^{13}$ représente un groupe alkyle en $C_{1-7}$,

avec la restriction que si $R^{4a}$ représente le groupe amino, $R^{3a}$ est différent d'un atome d'hydrogène,
ainsi que leurs sels pharmaceutiquement acceptables.

**9.** Composés de formule $Ia_1$ selon la revendication 8, dans lesquels $R^{3a}$ représente un atome d'hydrogène ou le groupe amino ou méthylamino ; $R^{4a}$ représente le groupe amino ou méthylamino ;
ainsi que leurs sels pharmaceutiquement acceptables.

**10.** Composé de formule $Ia_2$ selon la revendication 8, dans lequel

$R^{1a}$ représente le groupe 3-trifluorométhyle,
$R^{13}$ représente le groupe méthyle,

ainsi que ses sels pharmaceutiquement acceptables.

**11.** Composés de formules $Ib_1$ et $Ib_2$ choisis dans le groupe des composés de formule Ib de la revendication 4,

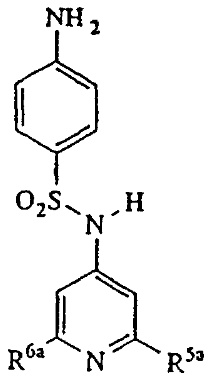

Ib$_1$ ,

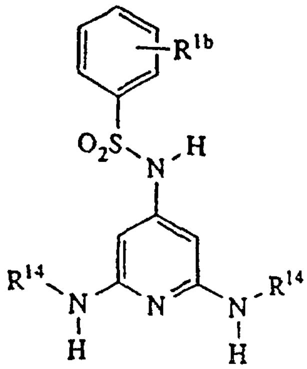

Ib$_2$

formules dans lesquelles

$R^{1b}$ représente un groupe 3-halogéno, 4-halogéno ou 3-trifluorométhyle ;
$R^{5a}$ représente un atome d'hydrogène ou d'halogène, ou un groupe alkyl($C_{1-7}$)amino ou dialkyl($C_{1-7}$)amino ;
$R^{6a}$ représente un atome d'halogène, ou un groupe alkyle en $C_{1-7}$, $CF_3$, alkyl($C_{1-7}$)amino ou dialkyl($C_{1-7}$)amino et
$R^{14}$ xreprésente un groupe alkyle en $C_{1-7}$,

avec la restriction que si $R^{6a}$ représente un atome d'halogène, $R^{5a}$ est différent d'un atome d'hydrogène, ainsi que leurs sels pharmaceutiquement acceptables.

**12.** Composés de formule Ib$_1$ selon la revendication 11, dans lesquels

$R^{5a}$ représente un atome d'hydrogène, de chlore ou de brome, ou le groupe méthylamino, éthylamino ou diméthylamino et
$R^{6a}$ représente un atome de brome ou le groupe méthyle, méthylamino, éthylamino ou diméthylamino ;

avec la restriction que si $R^{6a}$ représente un atome de brome, $R^{5a}$ est différent d'un atome d'hydrogène, ainsi que leurs sels pharmaceutiquement acceptables.

**13.** Composés de formule Ib$_2$ selon la revendication 11, dans lesquels

$R^{1b}$ représente un groupe 4-chloro, 3-chloro ou 3-trifluorométhyle ;
$R^{14}$ représente le groupe méthyle ;

ainsi que leurs sels pharmaceutiquement acceptables.

**14.** Composés de formules Ic$_1$ et Ic$_2$ choisis dans le groupe des composés de formule Ic de la revendication 5,

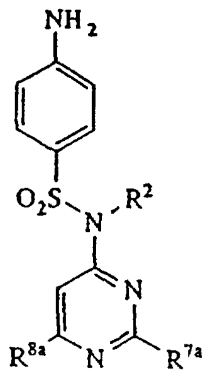 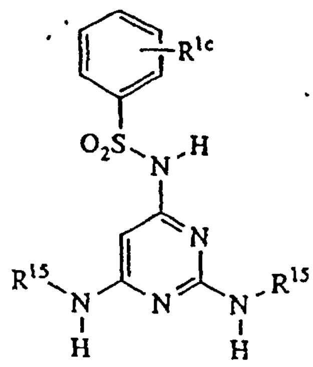

Ic$_1$ , Ic$_2$

formules dans lesquelles

- $R^{1c}$ représente un atome d'hydrogène ou un groupe 4-halogéno, 4-alkyle inférieur, 3-halogéno ou 3-trifluorométhyle ;
- $R^2$ représente un atome d'hydrogène ou un groupe alkyle en $C_{1-7}$ ;
- $R^{7a}$ représente un groupe amino, alkyl($C_{1-7}$)amino, dialkyl($C_{1-7}$)amino, mercapto ; pyrrolidin-1-yle ou azétidin-1-yle ;
- $R^{8a}$ représente un groupe amino, alkyl($C_{1-7}$)amino, dialkyl($C_{1-7}$)amino, benzylamino, alcoxy en $C_{1-7}$, pyrrolidin-1-yle ou azétidin-1-yle et
- $R^{15}$ représente un groupe alkyle en $C_{1-7}$,

avec la restriction que si $R^{7a}$ représente un groupe dialkyl($C_{1-7}$)amino, $R^{8a}$ est différent d'un groupe alcoxy en $C_{1-7}$ et d'un groupe dialkyl($C_{1-7}$)amino,
ainsi que leurs sels pharmaceutiquement acceptables.

**15.** Composés de formules Ic$_1$ selon la revendication 14, dans lesquels

- $R^2$ représente un atome d'hydrogène ou le groupe méthyle ;
- $R^{7a}$ représente un groupe amino, méthylamino, éthylamino, propylamino, iso-propylamino, diméthylamino, mercapto, pyrrolidin-1-yle ou azétidin-1-yle ;
- $R^{8a}$ représente un groupe amino, méthylamino, éthylamino, propylamino, iso-propylamino, diméthylamino, benzylamino, méthoxy, pyrrolidin-1-yle ou azétidin-1-yle,

avec la restriction que si $R^{7a}$ représente le groupe diméthylamino, $R^{8a}$ est différent des groupes diméthylamino et méthoxy,
ainsi que leurs sels pharmaceutiquement acceptables.

**16.** Composés de formule Ic$_2$ selon la revendication 11, dans lesquels

- $R^{1c}$ représente un atome d'hydrogène ou un groupe 4-fluoro, 4-chloro, 4-méthyle, 4-tert-butyle, 3-chloro ou 3-trifluorométhyle ;
- $R^{15}$ représente le groupe méthyle ;

ainsi que leurs sels pharmaceutiquement acceptables.

**17.** Composés de formules Id$_1$ et Id$_2$ choisis dans le groupe des composés de formule Id de la revendication 5,

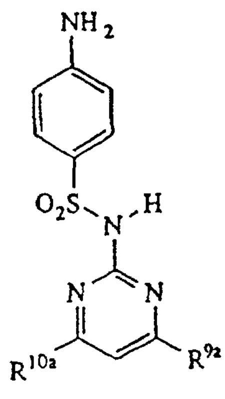 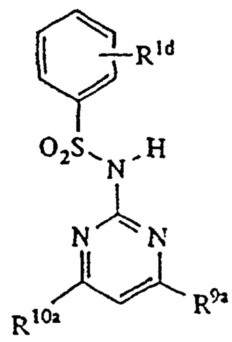

Id 1 , Id 2

formules dans lesquelles

| | |
|---|---|
| $R^{1d}$ | représente un groupe 3-trifluorométhyle, 4-trifluorométhyle, 3-halogéno ou 4-halogéno et |
| $R^{9a}$ et $R^{10a}$ | représentent des groupes alkyl($C_{1-7}$)amino, |

ainsi que leurs sels pharmaceutiquement acceptables.

**18.** Composés de formules le de la revendication 6

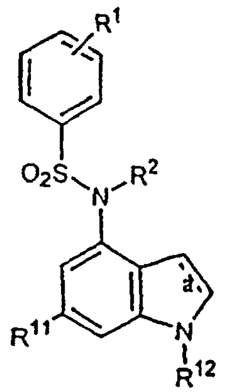

I e

dans laquelle $R^1$, $R^2$, $R^{11}$, $R^{12}$ et n ont les significations indiquées pour la formule I.

**19.** Médicaments, en particulier pour le traitement et/ou la prévention de troubles du système nerveux central, contenant un ou plusieurs composés selon l'une quelconque des revendications 8 à 18, et au moins un excipient thérapeutiquement inerte.

**20.** Procédé pour la préparation des composés de formule I selon les revendications 8 à 18, et de leurs sels, à partir d'un composé de formule II,

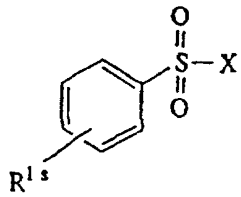

II

dans laquelle

$R^{1s}$ a la signification donnée pour $R^1$ ou représente un groupe amino protégé, et

X représente un atome d'halogène ou un groupe -NHY, et Y représente un métal alcalin, par exemple le sodium ou le potassium,

**caractérisé en ce qu'**on fait réagir un composé de formule II, dans laquelle X représente un atome d'halogène,

a) avec un composé de formule

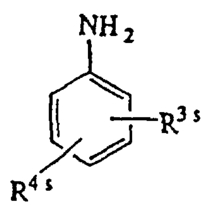

III a

dans laquelle

$R^{3s}$ représente un atome d'hydrogène ou d'halogène, ou un groupe alcoxy en $C_{1-7}$, un groupe amino protégé ou alkyl($C_{1-7}$)amino protégé et

$R^{4s}$ représente un groupe amino protégé ou alkyl($C_{1-7}$)amino protégé,

et on élimine les groupes protecteurs de fonction amino ; ou

b) avec un composé de formule

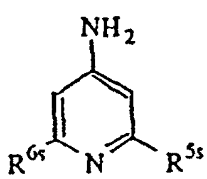

III b

dans laquelle

$R^{5s}$ représente un atome d'halogène et

$R^{6s}$ représente un atome d'halogène ou un groupe alkyle en $C_{1-7}$ ou $CF_3$,

et éventuellement on traite le produit de réaction par une alkyl($C_{1-7}$)amine ou dialkyl($C_{1-7}$)amine et on élimine le groupe protecteur de fonction amino ; ou

c) avec un composé de formule

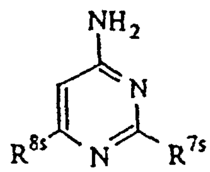 III c

dans laquelle

$R^{7s}$ représente le groupe mercapto et
$R^{8s}$ représente un groupe amino, alkyl($C_{1-7}$)amino, dialkyl($C_{1-7}$)amino, benzylamino, alcoxy en $C_{1-7}$, pyrrolidin-1-yle ou azétidin-1-yle,

et éventuellement on élimine le groupe protecteur de fonction amino, ou
d) on fait d'abord réagir un composé de formule II, dans lequel X représente un groupe X-NHY, avec un composé de formule

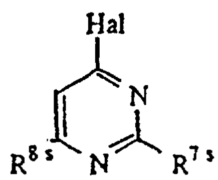 IIId

dans laquelle

$R^{7s}$ représente un groupe amino, alkyl($C_{1-7}$)amino, dialkyl($C_{1-7}$)amino, pyrrolidin-1-yle ou azétidin-1-yle et
$R^{8s}$ représente un atome d'halogène, et éventuellement on traite le produit de réaction par une alkyl($C_{1-7}$) amine, une dialkyl($C_{1-7}$)amine, l'azétidine, la pyrrolidine ou un alcoolate ; ou

e) on fait réagir un composé de formule II, dans laquelle X représente un atome d'halogène, avec un composé de formule

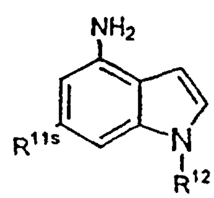

IIIe

dans laquelle
$R^{11s}$ représente un atome d'hydrogène ou d'halogène, et $R^{12}$ représente un atome d'hydrogène ou un groupe alkyle en $C_{1-7}$, et ensuite éventuellement on le réduit ensuite en un composé de formule Ie, dans laquelle a ne représente pas une double liaison, ou en ce que
f) on fait réagir avec une alkyl($C_{1-7}$)amine un composé de formule

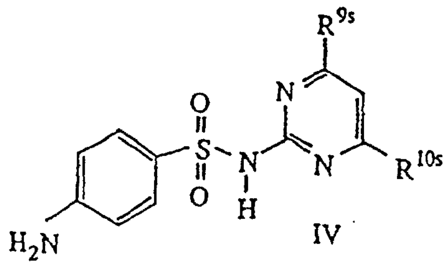

IV

dans laquelle
$R^{9s}$ et $R^{10s}$ représentent un groupe alcoxy en $C_{1-7}$, ou
g) on fait réagir avec une alkyl($C_{1-7}$)amine une sulfadiméthoxine de formule V

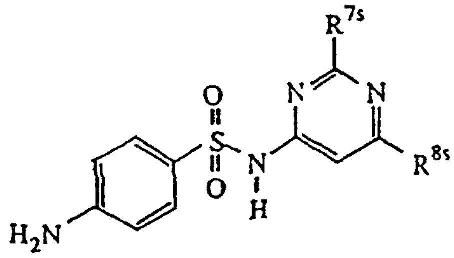

V

dans laquelle
$R^{7s}$ et $R^{8s}$ représentent un groupe alcoxy en $C_{1-7}$, et
h) si on le désire, on convertit un composé de formule générale I en un sel pharmaceutiquement acceptable.

**21.** Composés selon l'une quelconque des revendications 8 à 18, pour utilisation en tant que substances actives thérapeutiques, en particulier pour combattre ou prévenir des troubles du système nerveux central.

**22.** Utilisation des composés selon l'une quelconque des revendications 8 à 18, pour la fabrication d'un médicament destiné à combattre ou prévenir des troubles du système nerveux central.

**23.** Utilisation selon la revendication 22, pour combattre ou prévenir des troubles du système nerveux central, tels que les psychoses, la schizophrénie, les psychoses maniacodépressives, les dépressions, les troubles neurologiques, les troubles de la mémoire, la maladie de Parkinson, la sclérose latérale amyotrophique, la maladie d'Alzheimer et la chorée de Huntington.